# EUROPEAN PATENT APPLICATION

(11) **EP 2 295 565 A1**
(43) Date of publication of application: **16.03.2011**
(21) Application number: 09758461.9
(22) Date of filing: 08.06.2009
(51) Int. Cl.: C12N 15/09, C07K 19/00, C12N 1/15, C12N 1/19, C12N 5/10, C12P 21/02

(54) **POLYNUCLEOTIDE FOR RELEASING RECOMBINANT PROTEIN TO THE OUTSIDE OF EUKARYOTIC CELL**

(30) Priority: 06.06.2008 JP 2008149275
(71) Applicant: Saito, Saburo, Yokohama-shi, Kanagawa 223-0065 (JP); Akiyama, Nobutake, Tokyo 113-0031 (JP); Oriental Yeast Co., Ltd., Tokyo 174-8505 (JP)
(72) Inventor: OHNO, Yuji, Tokyo 153-0064 (JP); SAITO, Saburo, Kanagawa 223-0065 (JP); AKIYAMA, Nobutake, Tokyo 113-0031 (JP)
(74) Representative: Denison, Christopher Marcus
(86) International application number: PCT/JP2009/060811
(87) International publication number: WO 2009/148194

(57) **Abstract**

This invention provides a method for efficiently producing a recombinant protein by allowing the recombinant protein to express in a eukaryotic cell and releasing the expressed recombinant protein to the outside of the cell. The invention provides a polynucleotide used for producing a recombinant protein in a host cell comprising a polynucleotide encoding a glycosylation sequence comprising a transitional endoplasmic reticulum signal sequence and the sequence represented by: Asn-X- (Thr/Ser) (wherein X is an amino acid other than proline) and a polynucleotide encoding a target protein, which would not be efficiently released to the outside of the cell even when a transitional endoplasmic reticulum signal sequence is fused. The polynucleotide releases the target protein to the outside of the host cell via sugar chain modification.

SEQUENCE LISTING

<110> Saburo Saito
      Nobutake Akiyama
      ORIENTAL YEAST Co., Ltd.

<120> A polynucleotide for releasing a expressed recombinant protein
      produced in an eukaryote cell outside the cell

<130> PH-4042-PCT

<150> JP 2008-149275
<151> 2008-06-06

<160> 96

<170> PatentIn version 3.4

<210> 1
<211> 60
<212> DNA
<213> Mus musculus

<400> 1
atgggtctca acccccagct agttgtcatc ctgctcttct ttctcgaatg taccaggagc      60

<210> 2
<211> 20
<212> PRT
<213> Mus musculus

<400> 2

Met Gly Leu Asn Pro Gln Leu Val Val Ile Leu Leu Phe Phe Leu Glu
1               5                   10                  15

Cys Thr Arg Ser
            20

<210> 3
<211> 60
<212> DNA
<213> Mus musculus

<400> 3
atgagaagga tgcttctgca cttgagtgtt ctgactctca gctgtgtctg ggccactgcc 60

<210> 4
<211> 20
<212> PRT
<213> Mus musculus

<400> 4

Met Arg Arg Met Leu Leu His Leu Ser Val Leu Thr Leu Ser Cys Val
1               5                   10                  15

Trp Ala Thr Ala
            20

<210> 5
<211> 72
<212> DNA
<213> Mus musculus

<400> 5
atgaagttcc tctctgcaag agacttccat ccagttgcct tcttgggact gatgctggtg 60

acaaccacgg cc 72

<210> 6
<211> 24
<212> PRT
<213> Mus musculus

<400> 6

Met Lys Phe Leu Ser Ala Arg Asp Phe His Pro Val Ala Phe Leu Gly
1               5                   10                  15

Leu Met Leu Val Thr Thr Thr Ala
            20

<210>  7
<211>  69
<212>  DNA
<213>  Mus musculus

<400>  7
atgtgtcaat cacgctacct cctctttttg gccacccttg ccctcctaaa ccacctcagt      60

ttggccagg                                                             69

<210>  8
<211>  23
<212>  PRT
<213>  Mus musculus

<400>  8

Met Cys Gln Ser Arg Tyr Leu Leu Phe Leu Ala Thr Leu Ala Leu Leu
1               5                   10                  15

Asn His Leu Ser Leu Ala Arg
            20

<210>  9
<211>  63
<212>  DNA
<213>  Mus musculus

<400>  9
atggcgctct gggtgactgc agtcctggct cttgcttgcc ttggtggtct cgccgcccca      60

ggg                                                                    63

<210>  10
<211>  21
<212>  PRT
<213>  Mus musculus

<400>  10

Met Ala Leu Trp Val Thr Ala Val Leu Ala Leu Ala Cys Leu Gly Gly
1               5                   10                  15

Leu Ala Ala Pro Gly
            20

<210>  11
<211>  69
<212>  DNA
<213>  Mus musculus

<400>  11
atgatcttcc acacaggaac aacgaagcct accctggtgc tgctttgctg tataggaacc      60

tggctggcc                                                              69

<210>  12
<211>  23
<212>  PRT
<213>  Mus musculus

<400>  12

Met Ile Phe His Thr Gly Thr Thr Lys Pro Thr Leu Val Leu Leu Cys
1               5                   10                  15

Cys Ile Gly Thr Trp Leu Ala
            20

<210> 13
<211> 102
<212> DNA
<213> Homo sapiens

<400> 13
atgcatccgc tcctcaatcc tctcctgttg gcactgggcc tcatggcgct tttgttgacc 60

acggtcattg ctctcacttg ccttggcggc tttgcctccc ca 102

<210> 14
<211> 34
<212> PRT
<213> Homo sapiens

<400> 14

Met His Pro Leu Leu Asn Pro Leu Leu Leu Ala Leu Gly Leu Met Ala
1               5                   10                  15

Leu Leu Leu Thr Thr Val Ile Ala Leu Thr Cys Leu Gly Gly Phe Ala
            20                  25                  30

Ser Pro

<210> 15
<211> 69
<212> DNA
<213> Homo sapiens

<400> 15
atggcctctc actcaggccc ctcgacgtct gtgctctttc tgttctgctg cctgggaggc 60

tggctggcc 69

<210> 16
<211> 23
<212> PRT
<213> Homo sapiens

<400> 16

Met Ala Ser His Ser Gly Pro Ser Thr Ser Val Leu Phe Leu Phe Cys
1               5                   10                  15

Cys Leu Gly Gly Trp Leu Ala
            20

<210> 17
<211> 96
<212> DNA
<213> Artificial

<220>
<223> Synthetic

<400> 17
atgggaggat ccaattacac taacaattat agtaacattt ctaacaacta tagcaattac      60

actaacaatt atagtaacat ttctaacaac tatagc                                96

<210> 18
<211> 43
<212> PRT
<213> Artificial

<220>
<223> Synthetic

<400> 18

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Asn Tyr Thr
            20                  25                  30

Asn Asn Tyr Ser Asn Ile Ser Asn Asn Tyr Ser
        35                  40

<210> 19
<211> 489
<212> DNA
<213> Mus musculus

<400> 19
atgatcttcc acacaggaac aacgaagcct accctggtgc tgctttgctg tataggaacc      60

tggctggcca cctgcagctt gtccttcggt gccccaatat cgaaggaaga cttaagaact     120

acaattgacc tcttgaaaca agagtctcag gatctttata acaactatag cataaagcag     180

gcatctggga tgtcagcaga cgaatcaata cagctgccgt gtttcagcct ggaccgggaa     240

gcattaacca acatctcggt catcatagca catctggaga aagtcaaagt gttgagcgag     300

aacacagtag atacttcttg ggtgataaga tggctaacaa acatcagctg tttcaaccca     360

ctgaatttaa acatttctgt gcctggaaat actgatgaat cctatgattg taaagtgttc     420

gtgcttacgg ttttaaagca gttctcaaac tgcatggcag aactgcaggc taaggacaat     480

actacatgc                                                              489

<210> 20
<211> 163
<212> PRT
<213> Mus musculus

<400> 20

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Gly Ala Pro
            20                  25                  30

Ile Ser Lys Glu Asp Leu Arg Thr Thr Ile Asp Leu Leu Lys Gln Glu
        35                  40                  45

Ser Gln Asp Leu Tyr Asn Asn Tyr Ser Ile Lys Gln Ala Ser Gly Met
    50                  55                  60

Ser Ala Asp Glu Ser Ile Gln Leu Pro Cys Phe Ser Leu Asp Arg Glu
65                  70                  75                  80

Ala Leu Thr Asn Ile Ser Val Ile Ile Ala His Leu Glu Lys Val Lys
                85                  90                  95

Val Leu Ser Glu Asn Thr Val Asp Thr Ser Trp Val Ile Arg Trp Leu
            100                 105                 110

Thr Asn Ile Ser Cys Phe Asn Pro Leu Asn Leu Asn Ile Ser Val Pro
        115                 120                 125

Gly Asn Thr Asp Glu Ser Tyr Asp Cys Lys Val Phe Val Leu Thr Val
    130                 135                 140

Leu Lys Gln Phe Ser Asn Cys Met Ala Glu Leu Gln Ala Lys Asp Asn
145                 150                 155                 160

Thr Thr Cys

<210> 21
<211> 492
<212> DNA
<213> Mus musculus

<400> 21
atggcctctc actcaggccc ctcgacgtct gtgctctttc tgttctgctg cctgggaggc 60

tggctggcct cccacacgtt gcccgtccgt ttactacgac caagtgatga tgtacagaaa 120

atagtcgagg aattacagtc cctctcgaag atgcttttga aagatgtgga ggaagagaag 180

ggcgtgctcg tgtcccagaa ttacacgctg ccgtgtctca gccctgacgc ccagccgcca 240

aacaacatcc acagcccagc catccgggca tatctcaaga caatcagaca gctagacaac 300

aaatctgtta ttgatgagat catagagcac ctcgacaaac tcatatttca agatgcacca 360

gaaacaaaca tttctgtgcc aacagacacc catgaatgta aacgcttcat cctgactatt 420

tctcaacagt tttcagagtg catggacctc gcactaaaat cattgacctc tggagcccaa 480

caggccacca ct 492

<210> 22
<211> 164
<212> PRT
<213> Homo sapiens

<400> 22

Met Ala Ser His Ser Gly Pro Ser Thr Ser Val Leu Phe Leu Phe Cys
1               5                   10                  15

Cys Leu Gly Gly Trp Leu Ala Ser His Thr Leu Pro Val Arg Leu Leu
            20                  25                  30

Arg Pro Ser Asp Asp Val Gln Lys Ile Val Glu Glu Leu Gln Ser Leu
        35                  40                  45

Ser Lys Met Leu Leu Lys Asp Val Glu Glu Glu Lys Gly Val Leu Val
    50                  55                  60

Ser Gln Asn Tyr Thr Leu Pro Cys Leu Ser Pro Asp Ala Gln Pro Pro
65                  70                  75                  80

Asn Asn Ile His Ser Pro Ala Ile Arg Ala Tyr Leu Lys Thr Ile Arg
                85                  90                  95

Gln Leu Asp Asn Lys Ser Val Ile Asp Glu Ile Ile Glu His Leu Asp
            100                 105                 110

Lys Leu Ile Phe Gln Asp Ala Pro Glu Thr Asn Ile Ser Val Pro Thr
        115                 120                 125

Asp Thr His Glu Cys Lys Arg Phe Ile Leu Thr Ile Ser Gln Gln Phe
    130                 135                 140

Ser Glu Cys Met Asp Leu Ala Leu Lys Ser Leu Thr Ser Gly Ala Gln
145                 150                 155                 160

Gln Ala Thr Thr

<210>  23
<211>  801
<212>  DNA
<213>  Mus musculus

<400>  23

atgagaccta gaatgaagta ttccaactcc aagatttccc cggcaaagtt cagcagcacc 60

gcaggcgaag ccctggtccc gccttgcaaa ataagaagat cccaacagaa gaccaaagaa 120

ttctgccatg tctactgcat gagactccgt tctggcctca ccataagaaa ggagactagt 180

tattttagga aagaacccac gaaaagatat tcactaaaat cgggtaccaa gcatgaagag 240

aacttctctg cctatccacg ggattctagg aagagatcct tgcttggcag tatccaagca 300

tttgctgcgt ctgttgacac attgagcatc caaggaactt cacttttaac acagtctcct 360

gcctccctga gtacatacaa tgaccaatct gttagttttg ttttggagaa tggatgttat 420

gtgatcaatg ttgacgactc tggaaaagac caagagcaag accaggtgct actacgctac 480

tatgagtctc cctgtcctgc aagtcaatca ggcgacggtg tggatgggaa gaaggtgatg 540

gtgaacatga gtcccatcaa agacacagac atctggctgc atgccaacga caaggactac 600

tccgtggagc ttcaaagggg tgacgtctcg cctccggaac aggccttctt cgtccttcac 660

aaaaagtcct cggactttgt ttcatttgaa tgcaagaatc ttcctggcac ttacatagga 720

gtaaaagata accagctggc tctagtggag gagaaagatg agagctgcaa caatattatg 780

tttaagctct cgaaaatcta a 801

<210> 24
<211> 266
<212> PRT
<213> Mus musculus

<400> 24

Met Arg Pro Arg Met Lys Tyr Ser Asn Ser Lys Ile Ser Pro Ala Lys
1               5                   10                  15

Phe Ser Ser Thr Ala Gly Glu Ala Leu Val Pro Pro Cys Lys Ile Arg
            20                  25                  30

Arg Ser Gln Gln Lys Thr Lys Glu Phe Cys His Val Tyr Cys Met Arg
        35                  40                  45

Leu Arg Ser Gly Leu Thr Ile Arg Lys Glu Thr Ser Tyr Phe Arg Lys
    50                  55                  60

Glu Pro Thr Lys Arg Tyr Ser Leu Lys Ser Gly Thr Lys His Glu Glu
65                  70                  75                  80

Asn Phe Ser Ala Tyr Pro Arg Asp Ser Arg Lys Arg Ser Leu Leu Gly
                85                  90                  95

Ser Ile Gln Ala Phe Ala Ala Ser Val Asp Thr Leu Ser Ile Gln Gly
            100                 105                 110

Thr Ser Leu Leu Thr Gln Ser Pro Ala Ser Leu Ser Thr Tyr Asn Asp
        115                 120                 125

Gln Ser Val Ser Phe Val Leu Glu Asn Gly Cys Tyr Val Ile Asn Val
    130                 135                 140

Asp Asp Ser Gly Lys Asp Gln Glu Gln Asp Gln Val Leu Leu Arg Tyr
145                 150                 155                 160

Tyr Glu Ser Pro Cys Pro Ala Ser Gln Ser Gly Asp Gly Val Asp Gly
                165                 170                 175

Lys Lys Val Met Val Asn Met Ser Pro Ile Lys Asp Thr Asp Ile Trp
            180                 185                 190

Leu His Ala Asn Asp Lys Asp Tyr Ser Val Glu Leu Gln Arg Gly Asp
        195                 200                 205

Val Ser Pro Pro Glu Gln Ala Phe Phe Val Leu His Lys Lys Ser Ser
    210                 215                 220

Asp Phe Val Ser Phe Glu Cys Lys Asn Leu Pro Gly Thr Tyr Ile Gly
225                 230                 235                 240

Val Lys Asp Asn Gln Leu Ala Leu Val Glu Glu Lys Asp Glu Ser Cys
                245                 250                 255

Asn Asn Ile Met Phe Lys Leu Ser Lys Ile
            260                 265

<210> 25
<211> 41
<212> DNA
<213> Artificial

<220>
<223> Primer

<400> 25
tttgaattca gcgatccgcc atgatcttcc acacaggaac a 41

<210> 26
<211> 37
<212> DNA
<213> Artificial

<220>
<223> Primer

<400> 26
tttctcgagg ccctcgaagg acaggcactg ctgagga 37

<210> 27
<211> 574
<212> DNA
<213> Mus musculus

<400> 27
tttgaattca gcgatccgcc atgatcttcc acacaggaac aacgaagcct accctggtgc 60

tgctttgctg tataggaacc tggctggcca cctgcagctt gtccttcggt gccccaatat 120

cgaaggaaga cttaagaact acaattgacc tcttgaaaca agagtctcag gatctttata 180

acaactatag cataaagcag gcatctggga tgtcagcaga cgaatcaata cagctgccgt 240

gtttcagcct ggaccgggaa gcattaacca acatctcggt catcatagca catctggaga 300

aagtcaaagt gttgagcgag aacacagtag atacttcttg ggtgataaga tggctaacaa 360

acatcagctg tttcaaccca ctgaatttaa acatttctgt gcctggaaat actgatgaat 420

cctatgattg taaagtgttc gtgcttacgg ttttaaagca gttctcaaac tgcatggcag 480

aactgcaggc taaggacaat actacatgct gagtgatggg gggggggggg tgcagtgtcc 540

tcagcagtgc ctgtccttcg agggcctcga gaaa 574

<210> 28
<211> 35
<212> DNA
<213> Artificial

<220>
<223> Primer

<400> 28
tttctcgagg catgtagtat tgtccttagc ctgca 35

<210> 29

<211>  35
<212>  DNA
<213>  Artificial

<220>
<223>  Primer

<400>  29
agaattcttg atgggtctca acccccagct agttg                                35


<210>  30
<211>  33
<212>  DNA
<213>  Artificial

<220>
<223>  Primer

<400>  30
tggatcccga gtaatccatt tgcatgatgc tct                                  33


<210>  31
<211>  35
<212>  DNA
<213>  Artificial

<220>
<223>  Primer

<400>  31
agaattcgtc atgagaagga tgcttctgca cttga                                35


<210>  32
<211>  35
<212>  DNA
<213>  Artificial

<220>
<223>  Primer

<400> 32
tggatccgcc ttccattgcc cactctgtac tcatc 35

<210> 33
<211> 32
<212> DNA
<213> Artificial

<220>
<223> Primer

<400> 33
agaattcatg aagttcctct ctgcaagaga ct 32

<210> 34
<211> 35
<212> DNA
<213> Artificial

<220>
<223> Primer

<400> 34
tggatccggt ttgccgagta gatctcaaag tgact 35

<210> 35
<211> 34
<212> DNA
<213> Artificial

<220>
<223> Primer

<400> 35
agaattcagc atgtgtcaat cacgctacct cctc 34

<210> 36

<211> 35
<212> DNA
<213> Artificial

<220>
<223> Primer

<400> 36
tggatccggc ggagctcaga tagcccatca ccctg 35

<210> 37
<211> 34
<212> DNA
<213> Artificial

<220>
<223> Primer

<400> 37
agaattcttc atggcgctct gggtgactgc agtc 34

<210> 38
<211> 34
<212> DNA
<213> Artificial

<220>
<223> Primer

<400> 38
tggatccgaa ggggccgtgg cgaaacagtt gctt 34

<210> 39
<211> 24
<212> DNA
<213> Artificial

<220>
<223> Primer

<400> 39
agcttgaatt caaaggatcc aaac 24

<210> 40
<211> 24
<212> DNA
<213> Artificial

<220>
<223> Primer

<400> 40
tcgagtttgg atcctttgaa ttca 24

<210> 41
<211> 40
<212> DNA
<213> Artificial

<220>
<223> Primer

<400> 41
ggatcctttc tcgagatggt gagcaagggc gaggagctgt 40

<210> 42
<211> 37
<212> DNA
<213> Artificial

<220>
<223> Primer

<400> 42
aaagtcgacg agtccggact tgtacagctc gtccatg 37

<210> 43

<211> 852
<212> DNA
<213> Artificial

<220>
<223> Synthetic

<400> 43
aagcttgaat tcaaaggatc ctttctcgag atggtgagca agggcgagga gctgttcacc 60

ggggtggtgc ccatcctggt cgagctggac ggcgacgtaa acggccacaa gttcagcgtg 120

tccggcgagg gcgagggcga tgccacctac ggcaagctga ccctgaagtt catctgcacc 180

accggcaagc tgcccgtgcc ctggcccacc ctcgtgacca ccctgaccta cggcgtgcag 240

tgcttcagcc gctaccccga ccacatgaag cagcacgact tcttcaagtc cgccatgccc 300

gaaggctacg tccaggagcg caccatcttc ttcaaggacg acggcaacta caagacccgc 360

gccgaggtga agttcgaggg cgacaccctg gtgaaccgca tcgagctgaa gggcatcgac 420

ttcaaggagg acggcaacat cctggggcac aagctggagt acaactacaa cagccacaac 480

gtctatatca tggccgacaa gcagaagaac ggcatcaagg tgaacttcaa gatccgccac 540

aacatcgagg acggcagcgt gcagctcgcc gaccactacc agcagaacac ccccatcggc 600

gacggccccg tgctgctgcc cgacaaccac tacctgagca cccagtccgc cctgagcaaa 660

gaccccaacg agaagcgcga tcacatggtc ctgctggagt tcgtgaccgc cgccgggatc 720

actctcggca tggacgagct gtacaagtcc ggactcgtcg agtctagagg gcccttcgaa 780

caaaaactca tctcagaaga ggatctgaat atgcataccg gtcatcatca ccatcaccat 840

tgagtttaaa cc 852

<210> 44
<211> 270
<212> PRT

<213>  Artificial

<220>
<223>  Synthetic

<400>  44

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ser
225                 230                 235                 240

Gly Leu Val Glu Ser Arg Gly Pro Phe Glu Gln Lys Leu Ile Ser Glu
                245                 250                 255

Glu Asp Leu Asn Met His Thr Gly His His His His His His
            260                 265                 270

<210>  45
<211>  415
<212>  PRT
<213>  Artificial

<220>
<223>  Synthetic

<400>  45

Met Gly Leu Asn Pro Gln Leu Val Val Ile Leu Leu Phe Phe Leu Glu
1               5                   10                  15

Cys Thr Arg Ser His Ile His Gly Cys Asp Lys Asn His Leu Arg Glu
            20                  25                  30

Ile Ile Gly Ile Leu Asn Glu Val Thr Gly Glu Gly Thr Pro Cys Thr
        35                  40                  45

Glu Met Asp Val Pro Asn Val Leu Thr Ala Thr Lys Asn Thr Thr Glu
    50                  55                  60

Ser Glu Leu Val Cys Arg Ala Ser Lys Val Leu Arg Ile Phe Tyr Leu
65                  70                  75                  80

Lys His Gly Lys Thr Pro Cys Leu Lys Lys Asn Ser Ser Val Leu Met
                85                  90                  95

Glu Leu Gln Arg Leu Phe Arg Ala Phe Arg Cys Leu Asp Ser Ser Ile
            100                 105                 110

Ser Cys Thr Met Asn Glu Ser Lys Ser Thr Ser Leu Lys Asp Phe Leu
        115                 120                 125

Glu Ser Leu Lys Ser Ile Met Gln Met Asp Tyr Ser Gly Ser Phe Leu
    130                 135                 140

Glu Met Val Ser Lys Gly Glu Glu Leu Phe Thr Gly Val Val Pro Ile
145                 150                 155                 160

Leu Val Glu Leu Asp Gly Asp Val Asn Gly His Lys Phe Ser Val Ser

                165                 170                 175

Gly Glu Gly Glu Gly Asp Ala Thr Tyr Gly Lys Leu Thr Leu Lys Phe
            180                 185                 190

Ile Cys Thr Thr Gly Lys Leu Pro Val Pro Trp Pro Thr Leu Val Thr
        195                 200                 205

Thr Leu Thr Tyr Gly Val Gln Cys Phe Ser Arg Tyr Pro Asp His Met
    210                 215                 220

Lys Gln His Asp Phe Phe Lys Ser Ala Met Pro Glu Gly Tyr Val Gln
225                 230                 235                 240

Glu Arg Thr Ile Phe Phe Lys Asp Asp Gly Asn Tyr Lys Thr Arg Ala
                245                 250                 255

Glu Val Lys Phe Glu Gly Asp Thr Leu Val Asn Arg Ile Glu Leu Lys
            260                 265                 270

Gly Ile Asp Phe Lys Glu Asp Gly Asn Ile Leu Gly His Lys Leu Glu
        275                 280                 285

Tyr Asn Tyr Asn Ser His Asn Val Tyr Ile Met Ala Asp Lys Gln Lys
    290                 295                 300

Asn Gly Ile Lys Val Asn Phe Lys Ile Arg His Asn Ile Glu Asp Gly
305                 310                 315                 320

Ser Val Gln Leu Ala Asp His Tyr Gln Gln Asn Thr Pro Ile Gly Asp
                325                 330                 335

Gly Pro Val Leu Leu Pro Asp Asn His Tyr Leu Ser Thr Gln Ser Ala
            340                 345                 350

Leu Ser Lys Asp Pro Asn Glu Lys Arg Asp His Met Val Leu Leu Glu
        355                 360                 365

Phe Val Thr Ala Ala Gly Ile Thr Leu Gly Met Asp Glu Leu Tyr Lys
    370                 375                 380

Ser Gly Leu Val Glu Ser Arg Gly Pro Phe Glu Gln Lys Leu Ile Ser
385                 390                 395                 400

Glu Glu Asp Leu Asn Met His Thr Gly His His His His His His
                405                 410                 415

<210> 46
<211> 408
<212> PRT
<213> Artificial

<220>
<223> Synthetic

<400> 46

Met Arg Arg Met Leu Leu His Leu Ser Val Leu Thr Leu Ser Cys Val
1               5                   10                  15

Trp Ala Thr Ala Met Glu Ile Pro Met Ser Thr Val Val Lys Glu Thr
            20                  25                  30

Leu Thr Gln Leu Ser Ala His Arg Ala Leu Leu Thr Ser Asn Glu Thr
        35                  40                  45

Met Arg Leu Pro Val Pro Thr His Lys Asn His Gln Leu Cys Ile Gly
    50                  55                  60

Glu Ile Phe Gln Gly Leu Asp Ile Leu Lys Asn Gln Thr Val Arg Gly
65                  70                  75                  80

Gly Thr Val Glu Met Leu Phe Gln Asn Leu Ser Leu Ile Lys Lys Tyr
                85                  90                  95

Ile Asp Arg Gln Lys Glu Lys Cys Gly Glu Glu Arg Arg Arg Thr Arg
            100                 105                 110

Gln Phe Leu Asp Tyr Leu Gln Glu Phe Leu Gly Val Met Ser Thr Glu
        115                 120                 125

Trp Ala Met Glu Gly Gly Ser Phe Leu Glu Met Val Ser Lys Gly Glu
    130                 135                 140

Glu Leu Phe Thr Gly Val Val Pro Ile Leu Val Glu Leu Asp Gly Asp
145                 150                 155                 160

Val Asn Gly His Lys Phe Ser Val Ser Gly Glu Gly Glu Gly Asp Ala
                165                 170                 175

Thr Tyr Gly Lys Leu Thr Leu Lys Phe Ile Cys Thr Thr Gly Lys Leu
            180                 185                 190

Pro Val Pro Trp Pro Thr Leu Val Thr Thr Leu Thr Tyr Gly Val Gln
        195                 200                 205

Cys Phe Ser Arg Tyr Pro Asp His Met Lys Gln His Asp Phe Phe Lys

210                 215                 220

Ser Ala Met Pro Glu Gly Tyr Val Gln Glu Arg Thr Ile Phe Phe Lys
225                 230                 235                 240

Asp Asp Gly Asn Tyr Lys Thr Arg Ala Glu Val Lys Phe Glu Gly Asp
                245                 250                 255

Thr Leu Val Asn Arg Ile Glu Leu Lys Gly Ile Asp Phe Lys Glu Asp
            260                 265                 270

Gly Asn Ile Leu Gly His Lys Leu Glu Tyr Asn Tyr Asn Ser His Asn
        275                 280                 285

Val Tyr Ile Met Ala Asp Lys Gln Lys Asn Gly Ile Lys Val Asn Phe
    290                 295                 300

Lys Ile Arg His Asn Ile Glu Asp Gly Ser Val Gln Leu Ala Asp His
305                 310                 315                 320

Tyr Gln Gln Asn Thr Pro Ile Gly Asp Gly Pro Val Leu Leu Pro Asp
                325                 330                 335

Asn His Tyr Leu Ser Thr Gln Ser Ala Leu Ser Lys Asp Pro Asn Glu
            340                 345                 350

Lys Arg Asp His Met Val Leu Leu Glu Phe Val Thr Ala Ala Gly Ile
        355                 360                 365

Thr Leu Gly Met Asp Glu Leu Tyr Lys Ser Gly Leu Val Glu Ser Arg
    370                 375                 380

Gly Pro Phe Glu Gln Lys Leu Ile Ser Glu Glu Asp Leu Asn Met His
385                 390                 395                 400

Thr Gly His His His His His His
                405

<210>  47
<211>  486
<212>  PRT
<213>  Artificial

<220>
<223>  Synthetic

<400>  47

Met Lys Phe Leu Ser Ala Arg Asp Phe His Pro Val Ala Phe Leu Gly
1               5                   10                  15

Leu Met Leu Val Thr Thr Thr Ala Phe Pro Thr Ser Gln Val Arg Arg
            20                  25                  30

Gly Asp Phe Thr Glu Asp Thr Thr Pro Asn Arg Pro Val Tyr Thr Thr
        35                  40                  45

Ser Gln Val Gly Gly Leu Ile Thr His Val Leu Trp Glu Ile Val Glu
    50                  55                  60

Met Arg Lys Glu Leu Cys Asn Gly Asn Ser Asp Cys Met Asn Asn Asp
65                  70                  75                  80

Asp Ala Leu Ala Glu Asn Asn Leu Lys Leu Pro Glu Ile Gln Arg Asn
                85                  90                  95

Asp Gly Cys Tyr Gln Thr Gly Tyr Asn Gln Glu Ile Cys Leu Leu Lys
            100                 105                 110

Ile Ser Ser Gly Leu Leu Glu Tyr His Ser Tyr Leu Glu Tyr Met Lys
        115                 120                 125

Asn Asn Leu Lys Asp Asn Lys Lys Asp Lys Ala Arg Val Leu Gln Arg
    130                 135                 140

Asp Thr Glu Thr Leu Ile His Ile Phe Asn Gln Glu Val Lys Asp Leu
145                 150                 155                 160

His Lys Ile Val Leu Pro Thr Pro Ile Ser Asn Ala Leu Leu Thr Asp
                165                 170                 175

Lys Leu Glu Ser Gln Lys Glu Trp Leu Arg Thr Lys Thr Ile Gln Phe
            180                 185                 190

Ile Leu Lys Ser Leu Glu Glu Phe Leu Lys Val Thr Leu Arg Ser Thr
        195                 200                 205

Arg Gln Thr Gly Ser Phe Leu Glu Met Val Ser Lys Gly Glu Glu Leu
    210                 215                 220

Phe Thr Gly Val Val Pro Ile Leu Val Glu Leu Asp Gly Asp Val Asn
225                 230                 235                 240

Gly His Lys Phe Ser Val Ser Gly Glu Gly Glu Gly Asp Ala Thr Tyr
                245                 250                 255

Gly Lys Leu Thr Leu Lys Phe Ile Cys Thr Thr Gly Lys Leu Pro Val

            260                 265                 270

Pro Trp Pro Thr Leu Val Thr Thr Leu Thr Tyr Gly Val Gln Cys Phe
        275                 280                 285

Ser Arg Tyr Pro Asp His Met Lys Gln His Asp Phe Phe Lys Ser Ala
    290                 295                 300

Met Pro Glu Gly Tyr Val Gln Glu Arg Thr Ile Phe Phe Lys Asp Asp
305                 310                 315                 320

Gly Asn Tyr Lys Thr Arg Ala Glu Val Lys Phe Glu Gly Asp Thr Leu
                325                 330                 335

Val Asn Arg Ile Glu Leu Lys Gly Ile Asp Phe Lys Glu Asp Gly Asn
            340                 345                 350

Ile Leu Gly His Lys Leu Glu Tyr Asn Tyr Asn Ser His Asn Val Tyr
        355                 360                 365

Ile Met Ala Asp Lys Gln Lys Asn Gly Ile Lys Val Asn Phe Lys Ile
    370                 375                 380

Arg His Asn Ile Glu Asp Gly Ser Val Gln Leu Ala Asp His Tyr Gln
385                 390                 395                 400

Gln Asn Thr Pro Ile Gly Asp Gly Pro Val Leu Leu Pro Asp Asn His
                405                 410                 415

Tyr Leu Ser Thr Gln Ser Ala Leu Ser Lys Asp Pro Asn Glu Lys Arg
            420                 425                 430

Asp His Met Val Leu Leu Glu Phe Val Thr Ala Ala Gly Ile Thr Leu
        435                 440                 445

Gly Met Asp Glu Leu Tyr Lys Ser Gly Leu Val Glu Ser Arg Gly Pro
    450                 455                 460

Phe Glu Gln Lys Leu Ile Ser Glu Glu Asp Leu Asn Met His Thr Gly
465                 470                 475                 480

His His His His His His
                485

<210>  48
<211>  490
<212>  PRT
<213>  Artificial

<220>
<223>  Synthetic

<400>  48

Met Cys Gln Ser Arg Tyr Leu Leu Phe Leu Ala Thr Leu Ala Leu Leu
1               5                   10                  15

Asn His Leu Ser Leu Ala Arg Val Ile Pro Val Ser Gly Pro Ala Arg
            20                  25                  30

Cys Leu Ser Gln Ser Arg Asn Leu Leu Lys Thr Thr Asp Asp Met Val
        35                  40                  45

Lys Thr Ala Arg Glu Lys Leu Lys His Tyr Ser Cys Thr Ala Glu Asp
    50                  55                  60

Ile Asp His Glu Asp Ile Thr Arg Asp Gln Thr Ser Thr Leu Lys Thr
65                  70                  75                  80

Cys Leu Pro Leu Glu Leu His Lys Asn Glu Ser Cys Leu Ala Thr Arg
                85                  90                  95

Glu Thr Ser Ser Thr Thr Arg Gly Ser Cys Leu Pro Pro Gln Lys Thr
            100                 105                 110

Ser Leu Met Met Thr Leu Cys Leu Gly Ser Ile Tyr Glu Asp Leu Lys
        115                 120                 125

Met Tyr Gln Thr Glu Phe Gln Ala Ile Asn Ala Ala Leu Gln Asn His
    130                 135                 140

Asn His Gln Gln Ile Ile Leu Asp Lys Gly Met Leu Val Ala Ile Asp
145                 150                 155                 160

Glu Leu Met Gln Ser Leu Asn His Asn Gly Glu Thr Leu Arg Gln Lys
                165                 170                 175

Pro Pro Val Gly Glu Ala Asp Pro Tyr Arg Val Lys Met Lys Leu Cys
            180                 185                 190

Ile Leu Leu His Ala Phe Ser Thr Arg Val Val Thr Ile Asn Arg Val
        195                 200                 205

Met Gly Tyr Leu Ser Ser Ala Gly Ser Phe Leu Glu Met Val Ser Lys
    210                 215                 220

Gly Glu Glu Leu Phe Thr Gly Val Val Pro Ile Leu Val Glu Leu Asp

225                 230                 235                 240

Gly Asp Val Asn Gly His Lys Phe Ser Val Ser Gly Glu Gly Glu Gly
                245                 250                 255

Asp Ala Thr Tyr Gly Lys Leu Thr Leu Lys Phe Ile Cys Thr Thr Gly
            260                 265                 270

Lys Leu Pro Val Pro Trp Pro Thr Leu Val Thr Thr Leu Thr Tyr Gly
        275                 280                 285

Val Gln Cys Phe Ser Arg Tyr Pro Asp His Met Lys Gln His Asp Phe
    290                 295                 300

Phe Lys Ser Ala Met Pro Glu Gly Tyr Val Gln Glu Arg Thr Ile Phe
305                 310                 315                 320

Phe Lys Asp Asp Gly Asn Tyr Lys Thr Arg Ala Glu Val Lys Phe Glu
                325                 330                 335

Gly Asp Thr Leu Val Asn Arg Ile Glu Leu Lys Gly Ile Asp Phe Lys
            340                 345                 350

Glu Asp Gly Asn Ile Leu Gly His Lys Leu Glu Tyr Asn Tyr Asn Ser
        355                 360                 365

His Asn Val Tyr Ile Met Ala Asp Lys Gln Lys Asn Gly Ile Lys Val
    370                 375                 380

Asn Phe Lys Ile Arg His Asn Ile Glu Asp Gly Ser Val Gln Leu Ala
385                 390                 395                 400

Asp His Tyr Gln Gln Asn Thr Pro Ile Gly Asp Gly Pro Val Leu Leu
                405                 410                 415

Pro Asp Asn His Tyr Leu Ser Thr Gln Ser Ala Leu Ser Lys Asp Pro
            420                 425                 430

Asn Glu Lys Arg Asp His Met Val Leu Leu Glu Phe Val Thr Ala Ala
        435                 440                 445

Gly Ile Thr Leu Gly Met Asp Glu Leu Tyr Lys Ser Gly Leu Val Glu
    450                 455                 460

Ser Arg Gly Pro Phe Glu Gln Lys Leu Ile Ser Glu Glu Asp Leu Asn
465                 470                 475                 480

Met His Thr Gly His His His His His His
                485                 490

<210> 49
<211> 406
<212> PRT
<213> Artificial

<220>
<223> Synthetic

<400> 49

Met Ala Leu Trp Val Thr Ala Val Leu Ala Leu Ala Cys Leu Gly Gly
1               5                   10                  15

Leu Ala Ala Pro Gly Pro Val Pro Arg Ser Val Ser Leu Pro Leu Thr
            20                  25                  30

Leu Lys Glu Leu Ile Glu Glu Leu Ser Asn Ile Thr Gln Asp Gln Thr
        35                  40                  45

Pro Leu Cys Asn Gly Ser Met Val Trp Ser Val Asp Leu Ala Ala Gly
    50                  55                  60

Gly Phe Cys Val Ala Leu Asp Ser Leu Thr Asn Ile Ser Asn Cys Asn
65                  70                  75                  80

Ala Ile Tyr Arg Thr Gln Arg Ile Leu His Gly Leu Cys Asn Arg Lys
                85                  90                  95

Ala Pro Thr Thr Val Ser Ser Leu Pro Asp Thr Lys Ile Glu Val Ala
            100                 105                 110

His Phe Ile Thr Lys Leu Leu Ser Tyr Thr Lys Gln Leu Phe Arg His
        115                 120                 125

Gly Pro Phe Gly Ser Phe Leu Glu Met Val Ser Lys Gly Glu Glu Leu
    130                 135                 140

Phe Thr Gly Val Val Pro Ile Leu Val Glu Leu Asp Gly Asp Val Asn
145                 150                 155                 160

Gly His Lys Phe Ser Val Ser Gly Glu Gly Glu Gly Asp Ala Thr Tyr
                165                 170                 175

Gly Lys Leu Thr Leu Lys Phe Ile Cys Thr Thr Gly Lys Leu Pro Val
            180                 185                 190

Pro Trp Pro Thr Leu Val Thr Thr Leu Thr Tyr Gly Val Gln Cys Phe

        195                 200                 205

Ser Arg Tyr Pro Asp His Met Lys Gln His Asp Phe Phe Lys Ser Ala
    210                 215                 220

Met Pro Glu Gly Tyr Val Gln Glu Arg Thr Ile Phe Phe Lys Asp Asp
225                 230                 235                 240

Gly Asn Tyr Lys Thr Arg Ala Glu Val Lys Phe Glu Gly Asp Thr Leu
                245                 250                 255

Val Asn Arg Ile Glu Leu Lys Gly Ile Asp Phe Lys Glu Asp Gly Asn
            260                 265                 270

Ile Leu Gly His Lys Leu Glu Tyr Asn Tyr Asn Ser His Asn Val Tyr
        275                 280                 285

Ile Met Ala Asp Lys Gln Lys Asn Gly Ile Lys Val Asn Phe Lys Ile
    290                 295                 300

Arg His Asn Ile Glu Asp Gly Ser Val Gln Leu Ala Asp His Tyr Gln
305                 310                 315                 320

Gln Asn Thr Pro Ile Gly Asp Gly Pro Val Leu Leu Pro Asp Asn His
                325                 330                 335

Tyr Leu Ser Thr Gln Ser Ala Leu Ser Lys Asp Pro Asn Glu Lys Arg
            340                 345                 350

Asp His Met Val Leu Leu Glu Phe Val Thr Ala Ala Gly Ile Thr Leu
        355                 360                 365

Gly Met Asp Glu Leu Tyr Lys Ser Gly Leu Val Glu Ser Arg Gly Pro
    370                 375                 380

Phe Glu Gln Lys Leu Ile Ser Glu Glu Asp Leu Asn Met His Thr Gly
385                 390                 395                 400

His His His His His His
                405

<210>  50
<211>  438
<212>  PRT
<213>  Artificial

<220>
<223>  Synthetic

<400>  50

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Gly Ala Pro
            20                  25                  30

Ile Ser Lys Glu Asp Leu Arg Thr Thr Ile Asp Leu Leu Lys Gln Glu
        35                  40                  45

Ser Gln Asp Leu Tyr Asn Asn Tyr Ser Ile Lys Gln Ala Ser Gly Met
    50                  55                  60

Ser Ala Asp Glu Ser Ile Gln Leu Pro Cys Phe Ser Leu Asp Arg Glu
65                  70                  75                  80

Ala Leu Thr Asn Ile Ser Val Ile Ile Ala His Leu Glu Lys Val Lys
                85                  90                  95

Val Leu Ser Glu Asn Thr Val Asp Thr Ser Trp Val Ile Arg Trp Leu
            100                 105                 110

Thr Asn Ile Ser Cys Phe Asn Pro Leu Asn Leu Asn Ile Ser Val Pro
        115                 120                 125

Gly Asn Thr Asp Glu Ser Tyr Asp Cys Lys Val Phe Val Leu Thr Val
    130                 135                 140

Leu Lys Gln Phe Ser Asn Cys Met Ala Glu Leu Gln Ala Lys Asp Asn
145                 150                 155                 160

Thr Thr Cys Gly Ser Phe Leu Glu Met Val Ser Lys Gly Glu Glu Leu
                165                 170                 175

Phe Thr Gly Val Val Pro Ile Leu Val Glu Leu Asp Gly Asp Val Asn
            180                 185                 190

Gly His Lys Phe Ser Val Ser Gly Glu Gly Glu Gly Asp Ala Thr Tyr
        195                 200                 205

Gly Lys Leu Thr Leu Lys Phe Ile Cys Thr Thr Gly Lys Leu Pro Val
    210                 215                 220

Pro Trp Pro Thr Leu Val Thr Thr Leu Thr Tyr Gly Val Gln Cys Phe
225                 230                 235                 240

Ser Arg Tyr Pro Asp His Met Lys Gln His Asp Phe Phe Lys Ser Ala

                245                 250                 255

Met Pro Glu Gly Tyr Val Gln Glu Arg Thr Ile Phe Phe Lys Asp Asp
            260                 265                 270

Gly Asn Tyr Lys Thr Arg Ala Glu Val Lys Phe Glu Gly Asp Thr Leu
        275                 280                 285

Val Asn Arg Ile Glu Leu Lys Gly Ile Asp Phe Lys Glu Asp Gly Asn
    290                 295                 300

Ile Leu Gly His Lys Leu Glu Tyr Asn Tyr Asn Ser His Asn Val Tyr
305                 310                 315                 320

Ile Met Ala Asp Lys Gln Lys Asn Gly Ile Lys Val Asn Phe Lys Ile
                325                 330                 335

Arg His Asn Ile Glu Asp Gly Ser Val Gln Leu Ala Asp His Tyr Gln
            340                 345                 350

Gln Asn Thr Pro Ile Gly Asp Gly Pro Val Leu Leu Pro Asp Asn His
        355                 360                 365

Tyr Leu Ser Thr Gln Ser Ala Leu Ser Lys Asp Pro Asn Glu Lys Arg
    370                 375                 380

Asp His Met Val Leu Leu Glu Phe Val Thr Ala Ala Gly Ile Thr Leu
385                 390                 395                 400

Gly Met Asp Glu Leu Tyr Lys Ser Gly Leu Val Glu Ser Arg Gly Pro
                405                 410                 415

Phe Glu Gln Lys Leu Ile Ser Glu Glu Asp Leu Asn Met His Thr Gly
            420                 425                 430

His His His His His His
        435

<210>  51
<211>  30
<212>  DNA
<213>  Artificial

<220>
<223>  Primer

<400>  51
gcaaatgggc ggtaggcgtg tacggtggga                                        30

<210>  52
<211>  34
<212>  DNA
<213>  Artificial

<220>
<223>  Primer

<400>  52
tttggatccg gtggccagcc aggttcctat acag                                   34

<210>  53
<211>  32
<212>  DNA
<213>  Artificial

<220>
<223>  Primer

<400>  53

ttttggatcc ataaagatcc tgagactctt gt                                    32

<210>  54
<211>  35
<212>  DNA
<213>  Artificial

<220>
<223>  Primer

<400>  54
tttggatccg ctatagttgt tataaagatc ctgag                                 35

<210>  55
<211>  35
<212>  DNA
<213>  Artificial

<220>
<223>  Primer

<400>  55
tttggatccg ctatagtcgt cataaagatc ctgag                                 35

<210>  56
<211>  40
<212>  DNA
<213>  Artificial

<220>
<223>  Primer

<400>  56
caagctgcag gtggccagcc aggttcctat acagcaaagc                            40

<210>  57
<211>  37
<212>  DNA

<213>  Artificial

<220>
<223>  Primer

<400>  57
cttgtcccat aattacacta acaattatag taacatt                                37

<210>  58
<211>  37
<212>  DNA
<213>  Artificial

<220>
<223>  Primer

<400>  58
acctggctgg ccacctgcag cttgtcccat aattaca                                37

<210>  59
<211>  40
<212>  DNA
<213>  Artificial

<220>
<223>  Primer

<400>  59
agaaaggatc cgctatagtt gttagaaatg ttactataat                             40

<210>  60
<211>  299
<212>  PRT
<213>  Artificial

<220>
<223>  Synthetic

<400>  60

Met Ile Phe His Thr Gly Thr Thr Lys Pro Thr Leu Val Leu Leu Cys
1               5                   10                  15

Cys Ile Gly Thr Trp Leu Ala Thr Gly Ser Phe Leu Glu Met Val Ser
            20                  25                  30

Lys Gly Glu Glu Leu Phe Thr Gly Val Val Pro Ile Leu Val Glu Leu
        35                  40                  45

Asp Gly Asp Val Asn Gly His Lys Phe Ser Val Ser Gly Glu Gly Glu
    50                  55                  60

Gly Asp Ala Thr Tyr Gly Lys Leu Thr Leu Lys Phe Ile Cys Thr Thr
65                  70                  75                  80

Gly Lys Leu Pro Val Pro Trp Pro Thr Leu Val Thr Thr Leu Thr Tyr
                85                  90                  95

Gly Val Gln Cys Phe Ser Arg Tyr Pro Asp His Met Lys Gln His Asp
            100                 105                 110

Phe Phe Lys Ser Ala Met Pro Glu Gly Tyr Val Gln Glu Arg Thr Ile
        115                 120                 125

Phe Phe Lys Asp Asp Gly Asn Tyr Lys Thr Arg Ala Glu Val Lys Phe
    130                 135                 140

Glu Gly Asp Thr Leu Val Asn Arg Ile Glu Leu Lys Gly Ile Asp Phe
145                 150                 155                 160

Lys Glu Asp Gly Asn Ile Leu Gly His Lys Leu Glu Tyr Asn Tyr Asn

                165                 170                 175

Ser His Asn Val Tyr Ile Met Ala Asp Lys Gln Lys Asn Gly Ile Lys
            180                 185                 190

Val Asn Phe Lys Ile Arg His Asn Ile Glu Asp Gly Ser Val Gln Leu
        195                 200                 205

Ala Asp His Tyr Gln Gln Asn Thr Pro Ile Gly Asp Gly Pro Val Leu
    210                 215                 220

Leu Pro Asp Asn His Tyr Leu Ser Thr Gln Ser Ala Leu Ser Lys Asp
225                 230                 235                 240

Pro Asn Glu Lys Arg Asp His Met Val Leu Leu Glu Phe Val Thr Ala
                245                 250                 255

Ala Gly Ile Thr Leu Gly Met Asp Glu Leu Tyr Lys Ser Gly Leu Val
            260                 265                 270

Glu Ser Arg Gly Pro Phe Glu Gln Lys Leu Ile Ser Glu Glu Asp Leu
        275                 280                 285

Asn Met His Thr Gly His His His His His His
    290                 295

<210> 61
<211> 328
<212> PRT
<213> Artificial

<220>
<223> Synthetic

<400> 61

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Gly Ala Pro
            20                  25                  30

Ile Ser Lys Glu Asp Leu Arg Thr Thr Ile Asp Leu Leu Lys Gln Glu
        35                  40                  45

Ser Gln Asp Leu Tyr Gly Ser Phe Leu Glu Met Val Ser Lys Gly Glu
    50                  55                  60

Glu Leu Phe Thr Gly Val Val Pro Ile Leu Val Glu Leu Asp Gly Asp
65                  70                  75                  80

Val Asn Gly His Lys Phe Ser Val Ser Gly Glu Gly Glu Gly Asp Ala
                85                  90                  95

Thr Tyr Gly Lys Leu Thr Leu Lys Phe Ile Cys Thr Thr Gly Lys Leu
            100                 105                 110

Pro Val Pro Trp Pro Thr Leu Val Thr Thr Leu Thr Tyr Gly Val Gln
        115                 120                 125

Cys Phe Ser Arg Tyr Pro Asp His Met Lys Gln His Asp Phe Phe Lys
    130                 135                 140

Ser Ala Met Pro Glu Gly Tyr Val Gln Glu Arg Thr Ile Phe Phe Lys
145                 150                 155                 160

Asp Asp Gly Asn Tyr Lys Thr Arg Ala Glu Val Lys Phe Glu Gly Asp
                    165                 170                 175

Thr Leu Val Asn Arg Ile Glu Leu Lys Gly Ile Asp Phe Lys Glu Asp
                180                 185                 190

Gly Asn Ile Leu Gly His Lys Leu Glu Tyr Asn Tyr Asn Ser His Asn
            195                 200                 205

Val Tyr Ile Met Ala Asp Lys Gln Lys Asn Gly Ile Lys Val Asn Phe
    210                 215                 220

Lys Ile Arg His Asn Ile Glu Asp Gly Ser Val Gln Leu Ala Asp His
225                 230                 235                 240

Tyr Gln Gln Asn Thr Pro Ile Gly Asp Gly Pro Val Leu Leu Pro Asp
                    245                 250                 255

Asn His Tyr Leu Ser Thr Gln Ser Ala Leu Ser Lys Asp Pro Asn Glu
                260                 265                 270

Lys Arg Asp His Met Val Leu Leu Glu Phe Val Thr Ala Ala Gly Ile
            275                 280                 285

Thr Leu Gly Met Asp Glu Leu Tyr Lys Ser Gly Leu Val Glu Ser Arg
    290                 295                 300

Gly Pro Phe Glu Gln Lys Leu Ile Ser Glu Glu Asp Leu Asn Met His
305                 310                 315                 320

Thr Gly His His His His His His

                325

<210> 62
<211> 332
<212> PRT
<213> Artificial

<220>
<223> Synthetic

<400> 62

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Gly Ala Pro
            20                  25                  30

Ile Ser Lys Glu Asp Leu Arg Thr Thr Ile Asp Leu Leu Lys Gln Glu
        35                  40                  45

Ser Gln Asp Leu Tyr Asn Asn Tyr Ser Gly Ser Phe Leu Glu Met Val
    50                  55                  60

Ser Lys Gly Glu Glu Leu Phe Thr Gly Val Val Pro Ile Leu Val Glu
65                  70                  75                  80

Leu Asp Gly Asp Val Asn Gly His Lys Phe Ser Val Ser Gly Glu Gly
                85                  90                  95

Glu Gly Asp Ala Thr Tyr Gly Lys Leu Thr Leu Lys Phe Ile Cys Thr
            100                 105                 110

Thr Gly Lys Leu Pro Val Pro Trp Pro Thr Leu Val Thr Thr Leu Thr

        115                 120                 125

Tyr Gly Val Gln Cys Phe Ser Arg Tyr Pro Asp His Met Lys Gln His
    130                 135                 140

Asp Phe Phe Lys Ser Ala Met Pro Glu Gly Tyr Val Gln Glu Arg Thr
145                 150                 155                 160

Ile Phe Phe Lys Asp Asp Gly Asn Tyr Lys Thr Arg Ala Glu Val Lys
                165                 170                 175

Phe Glu Gly Asp Thr Leu Val Asn Arg Ile Glu Leu Lys Gly Ile Asp
            180                 185                 190

Phe Lys Glu Asp Gly Asn Ile Leu Gly His Lys Leu Glu Tyr Asn Tyr
        195                 200                 205

Asn Ser His Asn Val Tyr Ile Met Ala Asp Lys Gln Lys Asn Gly Ile
    210                 215                 220

Lys Val Asn Phe Lys Ile Arg His Asn Ile Glu Asp Gly Ser Val Gln
225                 230                 235                 240

Leu Ala Asp His Tyr Gln Gln Asn Thr Pro Ile Gly Asp Gly Pro Val
                245                 250                 255

Leu Leu Pro Asp Asn His Tyr Leu Ser Thr Gln Ser Ala Leu Ser Lys
            260                 265                 270

Asp Pro Asn Glu Lys Arg Asp His Met Val Leu Leu Glu Phe Val Thr
        275                 280                 285

Ala Ala Gly Ile Thr Leu Gly Met Asp Glu Leu Tyr Lys Ser Gly Leu
    290                 295                 300

Val Glu Ser Arg Gly Pro Phe Glu Gln Lys Leu Ile Ser Glu Glu Asp
305                 310                 315                 320

Leu Asn Met His Thr Gly His His His His His His
                325                 330

<210>  63
<211>  332
<212>  PRT
<213>  Artificial

<220>
<223>  Synthetic

<400>  63

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Gly Ala Pro
            20                  25                  30

Ile Ser Lys Glu Asp Leu Arg Thr Thr Ile Asp Leu Leu Lys Gln Glu
        35                  40                  45

Ser Gln Asp Leu Tyr Asp Asp Tyr Ser Gly Ser Phe Leu Glu Met Val
    50                  55                  60

Ser Lys Gly Glu Glu Leu Phe Thr Gly Val Val Pro Ile Leu Val Glu
65                  70                  75                  80

Leu Asp Gly Asp Val Asn Gly His Lys Phe Ser Val Ser Gly Glu Gly
                85                  90                  95

Glu Gly Asp Ala Thr Tyr Gly Lys Leu Thr Leu Lys Phe Ile Cys Thr
            100                 105                 110

Thr Gly Lys Leu Pro Val Pro Trp Pro Thr Leu Val Thr Thr Leu Thr
        115                 120                 125

Tyr Gly Val Gln Cys Phe Ser Arg Tyr Pro Asp His Met Lys Gln His
    130                 135                 140

Asp Phe Phe Lys Ser Ala Met Pro Glu Gly Tyr Val Gln Glu Arg Thr
145                 150                 155                 160

Ile Phe Phe Lys Asp Asp Gly Asn Tyr Lys Thr Arg Ala Glu Val Lys
                165                 170                 175

Phe Glu Gly Asp Thr Leu Val Asn Arg Ile Glu Leu Lys Gly Ile Asp
            180                 185                 190

Phe Lys Glu Asp Gly Asn Ile Leu Gly His Lys Leu Glu Tyr Asn Tyr
        195                 200                 205

Asn Ser His Asn Val Tyr Ile Met Ala Asp Lys Gln Lys Asn Gly Ile
    210                 215                 220

Lys Val Asn Phe Lys Ile Arg His Asn Ile Glu Asp Gly Ser Val Gln
225                 230                 235                 240

Leu Ala Asp His Tyr Gln Gln Asn Thr Pro Ile Gly Asp Gly Pro Val

245                 250                 255

Leu Leu Pro Asp Asn His Tyr Leu Ser Thr Gln Ser Ala Leu Ser Lys
            260                 265                 270

Asp Pro Asn Glu Lys Arg Asp His Met Val Leu Leu Glu Phe Val Thr
        275                 280                 285

Ala Ala Gly Ile Thr Leu Gly Met Asp Glu Leu Tyr Lys Ser Gly Leu
    290                 295                 300

Val Glu Ser Arg Gly Pro Phe Glu Gln Lys Leu Ile Ser Glu Glu Asp
305                 310                 315                 320

Leu Asn Met His Thr Gly His His His His His His
                325                 330

<210>  64
<211>  318
<212>  PRT
<213>  Artificial

<220>
<223>  Synthetic

<400>  64

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Asn Tyr Thr
            20                  25                  30

Asn Asn Tyr Ser Asn Ile Ser Asn Asn Tyr Ser Gly Ser Phe Leu Glu

        35                  40                  45

Met Val Ser Lys Gly Glu Glu Leu Phe Thr Gly Val Val Pro Ile Leu
    50                  55                  60

Val Glu Leu Asp Gly Asp Val Asn Gly His Lys Phe Ser Val Ser Gly
65                  70                  75                  80

Glu Gly Glu Gly Asp Ala Thr Tyr Gly Lys Leu Thr Leu Lys Phe Ile
                85                  90                  95

Cys Thr Thr Gly Lys Leu Pro Val Pro Trp Pro Thr Leu Val Thr Thr
            100                 105                 110

Leu Thr Tyr Gly Val Gln Cys Phe Ser Arg Tyr Pro Asp His Met Lys
        115                 120                 125

Gln His Asp Phe Phe Lys Ser Ala Met Pro Glu Gly Tyr Val Gln Glu
    130                 135                 140

Arg Thr Ile Phe Phe Lys Asp Asp Gly Asn Tyr Lys Thr Arg Ala Glu
145                 150                 155                 160

Val Lys Phe Glu Gly Asp Thr Leu Val Asn Arg Ile Glu Leu Lys Gly
                165                 170                 175

Ile Asp Phe Lys Glu Asp Gly Asn Ile Leu Gly His Lys Leu Glu Tyr
            180                 185                 190

Asn Tyr Asn Ser His Asn Val Tyr Ile Met Ala Asp Lys Gln Lys Asn
        195                 200                 205

Gly Ile Lys Val Asn Phe Lys Ile Arg His Asn Ile Glu Asp Gly Ser
    210                 215                 220

Val Gln Leu Ala Asp His Tyr Gln Gln Asn Thr Pro Ile Gly Asp Gly
225                 230                 235                 240

Pro Val Leu Leu Pro Asp Asn His Tyr Leu Ser Thr Gln Ser Ala Leu
                245                 250                 255

Ser Lys Asp Pro Asn Glu Lys Arg Asp His Met Val Leu Leu Glu Phe
            260                 265                 270

Val Thr Ala Ala Gly Ile Thr Leu Gly Met Asp Glu Leu Tyr Lys Ser
        275                 280                 285

Gly Leu Val Glu Ser Arg Gly Pro Phe Glu Gln Lys Leu Ile Ser Glu
    290                 295                 300

Glu Asp Leu Asn Met His Thr Gly His His His His His His
305                 310                 315

<210>  65
<211>  33
<212>  DNA
<213>  Artificial

<220>
<223>  Primer

<400>  65
aaaggatcca tggaggagcc gcagtcagat cct                                33

<210>  66

<211>  34
<212>  DNA
<213>  Artificial

<220>
<223>  Primer

<400>  66
tttctcgagg tctgagtcag gcccttctgt cttg                                    34


<210>  67
<211>  421
<212>  PRT
<213>  Homo sapiens

<400>  67

Met Glu Glu Pro Gln Ser Asp Pro Ser Val Glu Pro Pro Leu Ser Gln
1               5                   10                  15


Glu Thr Phe Ser Asp Leu Trp Lys Leu Leu Pro Glu Asn Asn Val Leu
            20                  25                  30


Ser Pro Leu Pro Ser Gln Ala Met Asp Asp Leu Met Leu Ser Pro Asp
        35                  40                  45


Asp Ile Glu Gln Trp Phe Thr Glu Asp Pro Gly Pro Asp Glu Ala Pro
    50                  55                  60


Arg Met Pro Glu Ala Ala Pro Pro Val Ala Pro Ala Pro Ala Ala Pro
65                  70                  75                  80


Thr Pro Ala Ala Pro Ala Pro Ala Pro Ser Trp Pro Leu Ser Ser Ser
                85                  90                  95

Val Pro Ser Gln Lys Thr Tyr Gln Gly Ser Tyr Gly Phe Arg Leu Gly
            100                 105                 110

Phe Leu His Ser Gly Thr Ala Lys Ser Val Thr Cys Thr Tyr Ser Pro
        115                 120                 125

Ala Leu Asn Lys Met Phe Cys Gln Leu Ala Lys Thr Cys Pro Val Gln
    130                 135                 140

Leu Trp Val Asp Ser Thr Pro Pro Pro Gly Thr Arg Val Arg Ala Met
145                 150                 155                 160

Ala Ile Tyr Lys Gln Ser Gln His Met Thr Glu Val Val Arg Arg Cys
                165                 170                 175

Pro His His Glu Arg Cys Ser Asp Ser Asp Gly Leu Ala Pro Pro Gln
            180                 185                 190

His Leu Ile Arg Val Glu Gly Asn Leu Arg Val Glu Tyr Leu Asp Asp
        195                 200                 205

Arg Asn Thr Phe Arg His Ser Val Val Val Pro Tyr Glu Pro Pro Glu
    210                 215                 220

Val Gly Ser Asp Cys Thr Thr Ile His Tyr Asn Tyr Met Cys Asn Ser
225                 230                 235                 240

Ser Cys Met Gly Gly Met Asn Arg Arg Pro Ile Leu Thr Ile Ile Thr
                245                 250                 255

Leu Glu Asp Ser Ser Gly Asn Leu Leu Gly Arg Asn Ser Phe Glu Val
            260                 265                 270

Arg Val Cys Ala Cys Pro Gly Arg Asp Arg Arg Thr Glu Glu Glu Asn
        275                 280                 285

Leu Arg Lys Lys Gly Glu Pro His His Glu Leu Pro Pro Gly Ser Thr
    290                 295                 300

Lys Arg Ala Leu Pro Asn Asn Thr Ser Ser Ser Pro Gln Pro Lys Lys
305                 310                 315                 320

Lys Pro Leu Asp Gly Glu Tyr Phe Thr Leu Gln Ile Arg Gly Arg Glu
                325                 330                 335

Arg Phe Glu Met Phe Arg Glu Leu Asn Glu Ala Leu Glu Leu Lys Asp
            340                 345                 350

Ala Gln Ala Gly Lys Glu Pro Gly Gly Ser Arg Ala His Ser Ser His
        355                 360                 365

Leu Lys Ser Lys Lys Gly Gln Ser Thr Ser Arg His Lys Lys Leu Met
    370                 375                 380

Phe Lys Thr Glu Gly Pro Asp Ser Asp Leu Glu Ser Arg Gly Pro Phe
385                 390                 395                 400

Glu Gln Lys Leu Ile Ser Glu Glu Asp Leu Asn Met His Thr Gly His
                405                 410                 415

His His His His His
            420

<210> 68
<211> 447
<212> PRT
<213> Artificial

<220>
<223> Synthetic

<400> 68

Met Ile Phe His Thr Gly Thr Thr Lys Pro Thr Leu Val Leu Leu Cys
1               5                   10                  15

Cys Ile Gly Thr Trp Leu Ala Thr Gly Ser Met Glu Glu Pro Gln Ser
            20                  25                  30

Asp Pro Ser Val Glu Pro Pro Leu Ser Gln Glu Thr Phe Ser Asp Leu
        35                  40                  45

Trp Lys Leu Leu Pro Glu Asn Asn Val Leu Ser Pro Leu Pro Ser Gln
    50                  55                  60

Ala Met Asp Asp Leu Met Leu Ser Pro Asp Asp Ile Glu Gln Trp Phe
65                  70                  75                  80

Thr Glu Asp Pro Gly Pro Asp Glu Ala Pro Arg Met Pro Glu Ala Ala
                85                  90                  95

Pro Pro Val Ala Pro Ala Pro Ala Ala Pro Thr Pro Ala Ala Pro Ala
            100                 105                 110

Pro Ala Pro Ser Trp Pro Leu Ser Ser Ser Val Pro Ser Gln Lys Thr
        115                 120                 125

Tyr Gln Gly Ser Tyr Gly Phe Arg Leu Gly Phe Leu His Ser Gly Thr
    130                 135                 140

Ala Lys Ser Val Thr Cys Thr Tyr Ser Pro Ala Leu Asn Lys Met Phe
145                 150                 155                 160

Cys Gln Leu Ala Lys Thr Cys Pro Val Gln Leu Trp Val Asp Ser Thr
                165                 170                 175

Pro Pro Pro Gly Thr Arg Val Arg Ala Met Ala Ile Tyr Lys Gln Ser
            180                 185                 190

Gln His Met Thr Glu Val Val Arg Arg Cys Pro His His Glu Arg Cys
        195                 200                 205

Ser Asp Ser Asp Gly Leu Ala Pro Pro Gln His Leu Ile Arg Val Glu
    210                 215                 220

Gly Asn Leu Arg Val Glu Tyr Leu Asp Asp Arg Asn Thr Phe Arg His
225                 230                 235                 240

Ser Val Val Val Pro Tyr Glu Pro Pro Glu Val Gly Ser Asp Cys Thr
                245                 250                 255

Thr Ile His Tyr Asn Tyr Met Cys Asn Ser Ser Cys Met Gly Gly Met
            260                 265                 270

Asn Arg Arg Pro Ile Leu Thr Ile Ile Thr Leu Glu Asp Ser Ser Gly
        275                 280                 285

Asn Leu Leu Gly Arg Asn Ser Phe Glu Val Arg Val Cys Ala Cys Pro
    290                 295                 300

Gly Arg Asp Arg Arg Thr Glu Glu Glu Asn Leu Arg Lys Lys Gly Glu
305                 310                 315                 320

Pro His His Glu Leu Pro Pro Gly Ser Thr Lys Arg Ala Leu Pro Asn
                325                 330                 335

Asn Thr Ser Ser Ser Pro Gln Pro Lys Lys Lys Pro Leu Asp Gly Glu
            340                 345                 350

Tyr Phe Thr Leu Gln Ile Arg Gly Arg Glu Arg Phe Glu Met Phe Arg
        355                 360                 365

Glu Leu Asn Glu Ala Leu Glu Leu Lys Asp Ala Gln Ala Gly Lys Glu
    370                 375                 380

Pro Gly Gly Ser Arg Ala His Ser Ser His Leu Lys Ser Lys Lys Gly
385                 390                 395                 400

Gln Ser Thr Ser Arg His Lys Lys Leu Met Phe Lys Thr Glu Gly Pro
                405                 410                 415

Asp Ser Asp Leu Glu Ser Arg Gly Pro Phe Glu Gln Lys Leu Ile Ser
            420                 425                 430

Glu Glu Asp Leu Asn Met His Thr Gly His His His His His His
        435                 440                 445

<210>  69
<211>  480
<212>  PRT
<213>  Artificial

<220>
<223>  Synthetic

<400>  69

Met Ile Phe His Thr Gly Thr Thr Lys Pro Thr Leu Val Leu Leu Cys
1               5                   10                  15

Cys Ile Gly Thr Trp Leu Ala Thr Cys Ser Leu Ser Phe Gly Ala Pro
            20                  25                  30

Ile Ser Lys Glu Asp Leu Arg Thr Thr Ile Asp Leu Leu Lys Gln Glu
        35                  40                  45

Ser Gln Asp Leu Tyr Asn Asn Tyr Ser Gly Ser Met Glu Glu Pro Gln
    50                  55                  60

Ser Asp Pro Ser Val Glu Pro Pro Leu Ser Gln Glu Thr Phe Ser Asp
65                  70                  75                  80

Leu Trp Lys Leu Leu Pro Glu Asn Asn Val Leu Ser Pro Leu Pro Ser
                85                  90                  95

Gln Ala Met Asp Asp Leu Met Leu Ser Pro Asp Asp Ile Glu Gln Trp
            100                 105                 110

Phe Thr Glu Asp Pro Gly Pro Asp Glu Ala Pro Arg Met Pro Glu Ala
        115                 120                 125

Ala Pro Pro Val Ala Pro Ala Pro Ala Ala Pro Thr Pro Ala Ala Pro
    130                 135                 140

Ala Pro Ala Pro Ser Trp Pro Leu Ser Ser Ser Val Pro Ser Gln Lys
145                 150                 155                 160

Thr Tyr Gln Gly Ser Tyr Gly Phe Arg Leu Gly Phe Leu His Ser Gly
                165                 170                 175

Thr Ala Lys Ser Val Thr Cys Thr Tyr Ser Pro Ala Leu Asn Lys Met
            180                 185                 190

Phe Cys Gln Leu Ala Lys Thr Cys Pro Val Gln Leu Trp Val Asp Ser
        195                 200                 205

Thr Pro Pro Pro Gly Thr Arg Val Arg Ala Met Ala Ile Tyr Lys Gln
    210                 215                 220

Ser Gln His Met Thr Glu Val Val Arg Arg Cys Pro His His Glu Arg
225                 230                 235                 240

Cys Ser Asp Ser Asp Gly Leu Ala Pro Pro Gln His Leu Ile Arg Val
                245                 250                 255

Glu Gly Asn Leu Arg Val Glu Tyr Leu Asp Asp Arg Asn Thr Phe Arg
            260                 265                 270

His Ser Val Val Val Pro Tyr Glu Pro Pro Glu Val Gly Ser Asp Cys
        275                 280                 285

Thr Thr Ile His Tyr Asn Tyr Met Cys Asn Ser Ser Cys Met Gly Gly
    290                 295                 300

Met Asn Arg Arg Pro Ile Leu Thr Ile Ile Thr Leu Glu Asp Ser Ser
305                 310                 315                 320

Gly Asn Leu Leu Gly Arg Asn Ser Phe Glu Val Arg Val Cys Ala Cys
                325                 330                 335

Pro Gly Arg Asp Arg Arg Thr Glu Glu Glu Asn Leu Arg Lys Lys Gly
            340                 345                 350

Glu Pro His His Glu Leu Pro Pro Gly Ser Thr Lys Arg Ala Leu Pro
        355                 360                 365

Asn Asn Thr Ser Ser Ser Pro Gln Pro Lys Lys Lys Pro Leu Asp Gly
    370                 375                 380

Glu Tyr Phe Thr Leu Gln Ile Arg Gly Arg Glu Arg Phe Glu Met Phe
385                 390                 395                 400

Arg Glu Leu Asn Glu Ala Leu Glu Leu Lys Asp Ala Gln Ala Gly Lys
                405                 410                 415

Glu Pro Gly Gly Ser Arg Ala His Ser Ser His Leu Lys Ser Lys Lys
            420                 425                 430

Gly Gln Ser Thr Ser Arg His Lys Lys Leu Met Phe Lys Thr Glu Gly
        435                 440                 445

Pro Asp Ser Asp Leu Glu Ser Arg Gly Pro Phe Glu Gln Lys Leu Ile
    450                 455                 460

Ser Glu Glu Asp Leu Asn Met His Thr Gly His His His His His His
465                 470                 475                 480

<210> 70
<211> 34
<212> DNA
<213> Artificial

<220>
<223> Primer

<400> 70
aaaaggatcc cggcagaatc atcgagaaac ctga 34

<210> 71
<211> 37
<212> DNA
<213> Artificial

<220>
<223> Primer

<400> 71
tttctcgagg attttcgaga gcttaaacat aatattg 37

<210> 72
<211> 36
<212> DNA
<213> Artificial

<220>
<223> Primer

<400> 72
aaaggatcca tccaaggaac ttcactttta acacag 36

<210> 73
<211> 294
<212> PRT
<213> Mus musculus

<400> 73

Met Arg Pro Arg Met Lys Tyr Ser Asn Ser Lys Ile Ser Pro Ala Lys
1               5                   10                  15

Phe Ser Ser Thr Ala Gly Glu Ala Leu Val Pro Pro Cys Lys Ile Arg
            20                  25                  30

Arg Ser Gln Gln Lys Thr Lys Glu Phe Cys His Val Tyr Cys Met Arg
        35                  40                  45

Leu Arg Ser Gly Leu Thr Ile Arg Lys Glu Thr Ser Tyr Phe Arg Lys
    50                  55                  60

Glu Pro Thr Lys Arg Tyr Ser Leu Lys Ser Gly Thr Lys His Glu Glu
65                  70                  75                  80

Asn Phe Ser Ala Tyr Pro Arg Asp Ser Arg Lys Arg Ser Leu Leu Gly
                85                  90                  95

Ser Ile Gln Ala Phe Ala Ala Ser Val Asp Thr Leu Ser Ile Gln Gly
            100                 105                 110

Thr Ser Leu Leu Thr Gln Ser Pro Ala Ser Leu Ser Thr Tyr Asn Asp
        115                 120                 125

Gln Ser Val Ser Phe Val Leu Glu Asn Gly Cys Tyr Val Ile Asn Val
    130                 135                 140

Asp Asp Ser Gly Lys Asp Gln Glu Gln Asp Gln Val Leu Leu Arg Tyr
145                 150                 155                 160

Tyr Glu Ser Pro Cys Pro Ala Ser Gln Ser Gly Asp Gly Val Asp Gly

165                 170                 175

Lys Lys Leu Met Val Asn Met Ser Pro Ile Lys Asp Thr Asp Ile Trp
            180                 185                 190

Leu His Ala Asn Asp Lys Asp Tyr Ser Val Glu Leu Gln Arg Gly Asp
        195                 200                 205

Val Ser Pro Pro Glu Gln Ala Phe Phe Val Leu His Lys Lys Ser Ser
    210                 215                 220

Asp Phe Val Ser Phe Glu Cys Lys Asn Leu Pro Gly Thr Tyr Ile Gly
225                 230                 235                 240

Val Lys Asp Asn Gln Leu Ala Leu Val Glu Glu Lys Asp Glu Ser Cys
                245                 250                 255

Asn Asn Ile Met Phe Lys Leu Ser Lys Ile Leu Glu Ser Arg Gly Pro
            260                 265                 270

Phe Glu Gln Lys Leu Ile Ser Glu Glu Asp Leu Asn Met His Thr Gly
        275                 280                 285

His His His His His His
    290

<210>  74
<211>  211
<212>  PRT
<213>  Artificial

<220>
<223>  Synthetic

<400> 74

Met Ile Phe His Thr Gly Thr Thr Lys Pro Thr Leu Val Leu Leu Cys
1               5                   10                  15

Cys Ile Gly Thr Trp Leu Ala Thr Gly Ser Ile Gln Gly Thr Ser Leu
            20                  25                  30

Leu Thr Gln Ser Pro Ala Ser Leu Ser Thr Tyr Asn Asp Gln Ser Val
        35                  40                  45

Ser Phe Val Leu Glu Asn Gly Cys Tyr Val Ile Asn Val Asp Asp Ser
    50                  55                  60

Gly Lys Asp Gln Glu Gln Asp Gln Val Leu Leu Arg Tyr Tyr Glu Ser
65                  70                  75                  80

Pro Cys Pro Ala Ser Gln Ser Gly Asp Gly Val Asp Gly Lys Lys Leu
                85                  90                  95

Met Val Asn Met Ser Pro Ile Lys Asp Thr Asp Ile Trp Leu His Ala
            100                 105                 110

Asn Asp Lys Asp Tyr Ser Val Glu Leu Gln Arg Gly Asp Val Ser Pro
        115                 120                 125

Pro Glu Gln Ala Phe Phe Val Leu His Lys Lys Ser Ser Asp Phe Val
    130                 135                 140

Ser Phe Glu Cys Lys Asn Leu Pro Gly Thr Tyr Ile Gly Val Lys Asp
145                 150                 155                 160

Asn Gln Leu Ala Leu Val Glu Glu Lys Asp Glu Ser Cys Asn Asn Ile
                165                 170                 175

Met Phe Lys Leu Ser Lys Ile Leu Glu Ser Arg Gly Pro Phe Glu Gln
            180                 185                 190

Lys Leu Ile Ser Glu Glu Asp Leu Asn Met His Thr Gly His His His
        195                 200                 205

His His His
    210

<210> 75
<211> 33
<212> DNA
<213> Artificial

<220>
<223> Primer

<400> 75
atggacaaca ccgaggacgt catcaaggag ttc 33

<210> 76
<211> 33
<212> DNA
<213> Artificial

<220>
<223> Primer

<400> 76
tttgtcgacc tgggagccgg agtggcgggc ctc 33

<210> 77

<211> 684
<212> DNA
<213> Artificial

<220>
<223> Synthetic

<400> 77
atggacaaca ccgaggacgt catcaaggag ttcatgcagt tcaaggtgcg catggagggc 60

tccgtgaacg gccactactt cgagatcgag ggcgagggcg agggcaagcc ctacgagggc 120

acccagaccg ccaagctgca ggtgaccaag ggcggccccc tgcccttcgc ctgggacatc 180

ctgtcccccc agttccagta cggctccaag gcctacgtga agcaccccgc cgacatcccc 240

gactacatga agctgtcctt ccccgagggc ttcacctggg agcgctccat gaacttcgag 300

gacggcggcg tggtggaggt gcagcaggac tcctccctgc aggacggcac cttcatctac 360

aaggtgaagt tcaagggcgt gaacttcccc gccgacggcc ccgtaatgca gaagaagact 420

gccggctggg agccctccac cgagaagctg tacccccagg acggcgtgct gaagggcgag 480

atctcccacg ccctgaagct gaaggacggc ggccactaca cctgcgactt caagaccgtg 540

tacaaggcca agaagcccgt gcagctgccc ggcaaccact acgtggactc caagctggac 600

atcaccaacc acaacgagga ctacaccgtg gtggagcagt acgagcacgc cgaggcccgc 660

cactccggct cccaggtcga caaa 684

<210> 78
<211> 39
<212> DNA
<213> Artificial

<220>
<223> Primer

<400> 78

gctatagttg ttagaaatgt tactataatt gttagtgta 39

<210> 79
<211> 39
<212> DNA
<213> Artificial

<220>
<223> Primer

<400> 79
tttgaattca tgggaggatc caattacact aacaattat 39

<210> 80
<211> 63
<212> DNA
<213> Artificial

<220>
<223> Primer

<400> 80
tttgaattca tgggaggatc caattacact aacaattata gtaacatttc taacaactat 60

agc 63

<210> 81
<211> 747
<212> DNA
<213> Artificial

<220>
<223> Synthetic

<400> 81
tttgaattca tgggaggatc caattacact aacaattata gtaacatttc taacaactat 60

agcatggaca acaccgagga cgtcatcaag gagttcatgc agttcaaggt gcgcatggag 120

ggctccgtga acggccacta cttcgagatc gagggcgagg gcgagggcaa gccctacgag 180

ggcacccaga ccgccaagct gcaggtgacc aagggcggcc ccctgccctt cgcctgggac 240

atcctgtccc cccagttcca gtacggctcc aaggcctacg tgaagcaccc cgccgacatc 300

cccgactaca tgaagctgtc cttccccgag ggcttcacct gggagcgctc catgaacttc 360

gaggacggcg gcgtggtgga ggtgcagcag gactcctccc tgcaggacgg caccttcatc 420

tacaaggtga agttcaaggg cgtgaacttc cccgccgacg gccccgtaat gcagaagaag 480

actgccggct gggagccctc caccgagaag ctgtaccccc aggacggcgt gctgaagggc 540

gagatctccc acgccctgaa gctgaaggac ggcggccact acacctgcga cttcaagacc 600

gtgtacaagg ccaagaagcc cgtgcagctg cccggcaacc actacgtgga ctccaagctg 660

gacatcacca accacaacga ggactacacc gtggtggagc agtacgagca cgccgaggcc 720

cgccactccg gctcccaggt cgacaaa 747

<210> 82
<211> 39
<212> DNA
<213> Artificial

<220>
<223> Primer

<400> 82
atcctcgagc accaccacca ccaccactga gcggccgct 39

<210> 83
<211> 43
<212> DNA
<213> Artificial

<220>
<223> Primer

<400> 83
ctagagcggc cgctcagtgg tggtggtggt ggtgctcgag gat 43

<210> 84
<211> 251
<212> PRT
<213> Artificial

<220>
<223> Synthetic

<400> 84

Met Gly Gly Ser Asn Tyr Thr Asn Asn Tyr Ser Asn Ile Ser Asn Asn
1               5                   10                  15

Tyr Ser Met Asp Asn Thr Glu Asp Val Ile Lys Glu Phe Met Gln Phe
            20                  25                  30

Lys Val Arg Met Glu Gly Ser Val Asn Gly His Tyr Phe Glu Ile Glu
        35                  40                  45

Gly Glu Gly Glu Gly Lys Pro Tyr Glu Gly Thr Gln Thr Ala Lys Leu
    50                  55                  60

Gln Val Thr Lys Gly Gly Pro Leu Pro Phe Ala Trp Asp Ile Leu Ser
65                  70                  75                  80

Pro Gln Phe Gln Tyr Gly Ser Lys Ala Tyr Val Lys His Pro Ala Asp
                85                  90                  95

Ile Pro Asp Tyr Met Lys Leu Ser Phe Pro Glu Gly Phe Thr Trp Glu
            100                 105                 110

Arg Ser Met Asn Phe Glu Asp Gly Gly Val Val Glu Val Gln Gln Asp
        115                 120                 125

Ser Ser Leu Gln Asp Gly Thr Phe Ile Tyr Lys Val Lys Phe Lys Gly
    130                 135                 140

Val Asn Phe Pro Ala Asp Gly Pro Val Met Gln Lys Lys Thr Ala Gly
145                 150                 155                 160

Trp Glu Pro Ser Thr Glu Lys Leu Tyr Pro Gln Asp Gly Val Leu Lys
                165                 170                 175

Gly Glu Ile Ser His Ala Leu Lys Leu Lys Asp Gly Gly His Tyr Thr
            180                 185                 190

Cys Asp Phe Lys Thr Val Tyr Lys Ala Lys Lys Pro Val Gln Leu Pro
        195                 200                 205

Gly Asn His Tyr Val Asp Ser Lys Leu Asp Ile Thr Asn His Asn Glu
    210                 215                 220

Asp Tyr Thr Val Val Glu Gln Tyr Glu His Ala Glu Ala Arg His Ser
225                 230                 235                 240

Gly Ser Gln Val Glu His His His His His His
                245                 250

<210>  85
<211>  32
<212>  DNA
<213>  Artificial

<220>
<223>  Primer

<400>  85
tttggatccg ctcctggtac attcgagaaa ga                                    32


<210>  86
<211>  32
<212>  DNA
<213>  Artificial

<220>
<223>  Primer

<400>  86
tttggatccg gcagtggccc agacacagct ga                                    32


<210>  87
<211>  32
<212>  DNA
<213>  Artificial

<220>
<223>  Primer

<400>  87
tttggatccg gccgtggttg tcaccagcat ca                                    32


<210>  88
<211>  33
<212>  DNA
<213>  Artificial

<220>
<223>  Primer

<400>  88
tttggatccc ctggccaaac tgaggtggtt tag                                   33

<210> 89
<211> 33
<212> DNA
<213> Artificial

<220>
<223> Primer

<400> 89
tttggatccc cctggggcgg cgagaccacc aag 33


<210> 90
<211> 270
<212> PRT
<213> Artificial

<220>
<223> Synthetic

<400> 90

Met Gly Leu Asn Pro Gln Leu Val Val Ile Leu Leu Phe Phe Leu Glu
1               5                   10                  15


Cys Thr Arg Ser Gly Gly Ser Asn Tyr Thr Asn Asn Tyr Ser Asn Ile
            20                  25                  30


Ser Asn Asn Tyr Ser Met Asp Asn Thr Glu Asp Val Ile Lys Glu Phe
        35                  40                  45


Met Gln Phe Lys Val Arg Met Glu Gly Ser Val Asn Gly His Tyr Phe
    50                  55                  60


Glu Ile Glu Gly Glu Gly Glu Gly Lys Pro Tyr Glu Gly Thr Gln Thr
65                  70                  75                  80

Ala Lys Leu Gln Val Thr Lys Gly Gly Pro Leu Pro Phe Ala Trp Asp
                85                  90                  95

Ile Leu Ser Pro Gln Phe Gln Tyr Gly Ser Lys Ala Tyr Val Lys His
            100                 105                 110

Pro Ala Asp Ile Pro Asp Tyr Met Lys Leu Ser Phe Pro Glu Gly Phe
        115                 120                 125

Thr Trp Glu Arg Ser Met Asn Phe Glu Asp Gly Gly Val Val Glu Val
    130                 135                 140

Gln Gln Asp Ser Ser Leu Gln Asp Gly Thr Phe Ile Tyr Lys Val Lys
145                 150                 155                 160

Phe Lys Gly Val Asn Phe Pro Ala Asp Gly Pro Val Met Gln Lys Lys
                165                 170                 175

Thr Ala Gly Trp Glu Pro Ser Thr Glu Lys Leu Tyr Pro Gln Asp Gly
            180                 185                 190

Val Leu Lys Gly Glu Ile Ser His Ala Leu Lys Leu Lys Asp Gly Gly
        195                 200                 205

His Tyr Thr Cys Asp Phe Lys Thr Val Tyr Lys Ala Lys Lys Pro Val
    210                 215                 220

Gln Leu Pro Gly Asn His Tyr Val Asp Ser Lys Leu Asp Ile Thr Asn
225                 230                 235                 240

His Asn Glu Asp Tyr Thr Val Val Glu Gln Tyr Glu His Ala Glu Ala

                245                 250                 255

Arg His Ser Gly Ser Gln Val Glu His His His His His His
            260                 265                 270

<210> 91
<211> 269
<212> PRT
<213> Artificial

<220>
<223> Synthetic

<400> 91

Met Arg Arg Met Leu Leu His Leu Ser Val Leu Thr Leu Ser Cys Val
1               5                   10                  15

Trp Ala Thr Ala Gly Ser Asn Tyr Thr Asn Asn Tyr Ser Asn Ile Ser
            20                  25                  30

Asn Asn Tyr Ser Met Asp Asn Thr Glu Asp Val Ile Lys Glu Phe Met
        35                  40                  45

Gln Phe Lys Val Arg Met Glu Gly Ser Val Asn Gly His Tyr Phe Glu
    50                  55                  60

Ile Glu Gly Glu Gly Glu Gly Lys Pro Tyr Glu Gly Thr Gln Thr Ala
65                  70                  75                  80

Lys Leu Gln Val Thr Lys Gly Gly Pro Leu Pro Phe Ala Trp Asp Ile
                85                  90                  95

Leu Ser Pro Gln Phe Gln Tyr Gly Ser Lys Ala Tyr Val Lys His Pro

            100                 105                 110

Ala Asp Ile Pro Asp Tyr Met Lys Leu Ser Phe Pro Glu Gly Phe Thr
        115                 120                 125

Trp Glu Arg Ser Met Asn Phe Glu Asp Gly Gly Val Val Glu Val Gln
    130                 135                 140

Gln Asp Ser Ser Leu Gln Asp Gly Thr Phe Ile Tyr Lys Val Lys Phe
145                 150                 155                 160

Lys Gly Val Asn Phe Pro Ala Asp Gly Pro Val Met Gln Lys Lys Thr
                165                 170                 175

Ala Gly Trp Glu Pro Ser Thr Glu Lys Leu Tyr Pro Gln Asp Gly Val
            180                 185                 190

Leu Lys Gly Glu Ile Ser His Ala Leu Lys Leu Lys Asp Gly Gly His
        195                 200                 205

Tyr Thr Cys Asp Phe Lys Thr Val Tyr Lys Ala Lys Lys Pro Val Gln
    210                 215                 220

Leu Pro Gly Asn His Tyr Val Asp Ser Lys Leu Asp Ile Thr Asn His
225                 230                 235                 240

Asn Glu Asp Tyr Thr Val Val Glu Gln Tyr Glu His Ala Glu Ala Arg
                245                 250                 255

His Ser Gly Ser Gln Val Glu His His His His His His
            260                 265

<210> 92
<211> 273
<212> PRT
<213> Artificial

<220>
<223> Synthetic

<400> 92

Met Lys Phe Leu Ser Ala Arg Asp Phe His Pro Val Ala Phe Leu Gly
1               5                   10                  15

Leu Met Leu Val Thr Thr Thr Ala Gly Ser Asn Tyr Thr Asn Asn Tyr
            20                  25                  30

Ser Asn Ile Ser Asn Asn Tyr Ser Met Asp Asn Thr Glu Asp Val Ile
        35                  40                  45

Lys Glu Phe Met Gln Phe Lys Val Arg Met Glu Gly Ser Val Asn Gly
    50                  55                  60

His Tyr Phe Glu Ile Glu Gly Glu Gly Glu Gly Lys Pro Tyr Glu Gly
65                  70                  75                  80

Thr Gln Thr Ala Lys Leu Gln Val Thr Lys Gly Gly Pro Leu Pro Phe
                85                  90                  95

Ala Trp Asp Ile Leu Ser Pro Gln Phe Gln Tyr Gly Ser Lys Ala Tyr
            100                 105                 110

Val Lys His Pro Ala Asp Ile Pro Asp Tyr Met Lys Leu Ser Phe Pro
        115                 120                 125

Glu Gly Phe Thr Trp Glu Arg Ser Met Asn Phe Glu Asp Gly Gly Val
    130                 135                 140

Val Glu Val Gln Gln Asp Ser Ser Leu Gln Asp Gly Thr Phe Ile Tyr
145                 150                 155                 160

Lys Val Lys Phe Lys Gly Val Asn Phe Pro Ala Asp Gly Pro Val Met
                165                 170                 175

Gln Lys Lys Thr Ala Gly Trp Glu Pro Ser Thr Glu Lys Leu Tyr Pro
            180                 185                 190

Gln Asp Gly Val Leu Lys Gly Glu Ile Ser His Ala Leu Lys Leu Lys
        195                 200                 205

Asp Gly Gly His Tyr Thr Cys Asp Phe Lys Thr Val Tyr Lys Ala Lys
    210                 215                 220

Lys Pro Val Gln Leu Pro Gly Asn His Tyr Val Asp Ser Lys Leu Asp
225                 230                 235                 240

Ile Thr Asn His Asn Glu Asp Tyr Thr Val Val Glu Gln Tyr Glu His
                245                 250                 255

Ala Glu Ala Arg His Ser Gly Ser Gln Val Glu His His His His His
            260                 265                 270

His

<210> 93

<211>  272
<212>  PRT
<213>  Artificial

<220>
<223>  Synthetic

<400>  93

Met Cys Gln Ser Arg Tyr Leu Leu Phe Leu Ala Thr Leu Ala Leu Leu
1               5                   10                  15

Asn His Leu Ser Leu Ala Arg Gly Ser Asn Tyr Thr Asn Asn Tyr Ser
            20                  25                  30

Asn Ile Ser Asn Asn Tyr Ser Met Asp Asn Thr Glu Asp Val Ile Lys
        35                  40                  45

Glu Phe Met Gln Phe Lys Val Arg Met Glu Gly Ser Val Asn Gly His
    50                  55                  60

Tyr Phe Glu Ile Glu Gly Glu Gly Glu Gly Lys Pro Tyr Glu Gly Thr
65                  70                  75                  80

Gln Thr Ala Lys Leu Gln Val Thr Lys Gly Gly Pro Leu Pro Phe Ala
                85                  90                  95

Trp Asp Ile Leu Ser Pro Gln Phe Gln Tyr Gly Ser Lys Ala Tyr Val
            100                 105                 110

Lys His Pro Ala Asp Ile Pro Asp Tyr Met Lys Leu Ser Phe Pro Glu
        115                 120                 125

Gly Phe Thr Trp Glu Arg Ser Met Asn Phe Glu Asp Gly Gly Val Val

130                 135                 140

Glu Val Gln Gln Asp Ser Ser Leu Gln Asp Gly Thr Phe Ile Tyr Lys
145                 150                 155                 160

Val Lys Phe Lys Gly Val Asn Phe Pro Ala Asp Gly Pro Val Met Gln
                165                 170                 175

Lys Lys Thr Ala Gly Trp Glu Pro Ser Thr Glu Lys Leu Tyr Pro Gln
            180                 185                 190

Asp Gly Val Leu Lys Gly Glu Ile Ser His Ala Leu Lys Leu Lys Asp
        195                 200                 205

Gly Gly His Tyr Thr Cys Asp Phe Lys Thr Val Tyr Lys Ala Lys Lys
    210                 215                 220

Pro Val Gln Leu Pro Gly Asn His Tyr Val Asp Ser Lys Leu Asp Ile
225                 230                 235                 240

Thr Asn His Asn Glu Asp Tyr Thr Val Val Glu Gln Tyr Glu His Ala
                245                 250                 255

Glu Ala Arg His Ser Gly Ser Gln Val Glu His His His His His His
            260                 265                 270

<210>  94
<211>  268
<212>  PRT
<213>  Artificial

<220>
<223>  Synthetic

<400> 94

Met Ala Leu Trp Val Thr Ala Val Leu Ala Leu Ala Cys Leu Gly Gly
1               5                   10                  15

Leu Ala Ala Pro Gly Asn Tyr Thr Asn Asn Tyr Ser Asn Ile Ser Asn
            20                  25                  30

Asn Tyr Ser Met Asp Asn Thr Glu Asp Val Ile Lys Glu Phe Met Gln
        35                  40                  45

Phe Lys Val Arg Met Glu Gly Ser Val Asn Gly His Tyr Phe Glu Ile
    50                  55                  60

Glu Gly Glu Gly Glu Gly Lys Pro Tyr Glu Gly Thr Gln Thr Ala Lys
65                  70                  75                  80

Leu Gln Val Thr Lys Gly Gly Pro Leu Pro Phe Ala Trp Asp Ile Leu
                85                  90                  95

Ser Pro Gln Phe Gln Tyr Gly Ser Lys Ala Tyr Val Lys His Pro Ala
            100                 105                 110

Asp Ile Pro Asp Tyr Met Lys Leu Ser Phe Pro Glu Gly Phe Thr Trp
        115                 120                 125

Glu Arg Ser Met Asn Phe Glu Asp Gly Gly Val Val Glu Val Gln Gln
    130                 135                 140

Asp Ser Ser Leu Gln Asp Gly Thr Phe Ile Tyr Lys Val Lys Phe Lys
145                 150                 155                 160

Gly Val Asn Phe Pro Ala Asp Gly Pro Val Met Gln Lys Lys Thr Ala
                165                 170                 175

Gly Trp Glu Pro Ser Thr Glu Lys Leu Tyr Pro Gln Asp Gly Val Leu
            180                 185                 190

Lys Gly Glu Ile Ser His Ala Leu Lys Leu Lys Asp Gly Gly His Tyr
        195                 200                 205

Thr Cys Asp Phe Lys Thr Val Tyr Lys Ala Lys Lys Pro Val Gln Leu
    210                 215                 220

Pro Gly Asn His Tyr Val Asp Ser Lys Leu Asp Ile Thr Asn His Asn
225                 230                 235                 240

Glu Asp Tyr Thr Val Val Glu Gln Tyr Glu His Ala Glu Ala Arg His
                245                 250                 255

Ser Gly Ser Gln Val Glu His His His His His His
            260                 265

<210>  95
<211>  273
<212>  PRT
<213>  Artificial

<220>
<223>  Synthetic

<400>  95

Met Ile Phe His Thr Gly Thr Thr Lys Pro Thr Leu Val Leu Leu Cys
1               5                   10                  15

Cys Ile Gly Thr Trp Leu Ala Thr Gly Ser Asn Tyr Thr Asn Asn Tyr
            20                  25                  30

Ser Asn Ile Ser Asn Asn Tyr Ser Met Asp Asn Thr Glu Asp Val Ile
        35                  40                  45

Lys Glu Phe Met Gln Phe Lys Val Arg Met Glu Gly Ser Val Asn Gly
    50                  55                  60

His Tyr Phe Glu Ile Glu Gly Glu Gly Glu Gly Lys Pro Tyr Glu Gly
65                  70                  75                  80

Thr Gln Thr Ala Lys Leu Gln Val Thr Lys Gly Gly Pro Leu Pro Phe
                85                  90                  95

Ala Trp Asp Ile Leu Ser Pro Gln Phe Gln Tyr Gly Ser Lys Ala Tyr
            100                 105                 110

Val Lys His Pro Ala Asp Ile Pro Asp Tyr Met Lys Leu Ser Phe Pro
        115                 120                 125

Glu Gly Phe Thr Trp Glu Arg Ser Met Asn Phe Glu Asp Gly Gly Val
    130                 135                 140

Val Glu Val Gln Gln Asp Ser Ser Leu Gln Asp Gly Thr Phe Ile Tyr
145                 150                 155                 160

Lys Val Lys Phe Lys Gly Val Asn Phe Pro Ala Asp Gly Pro Val Met
                165                 170                 175

Gln Lys Lys Thr Ala Gly Trp Glu Pro Ser Thr Glu Lys Leu Tyr Pro

180                 185                 190

Gln Asp Gly Val Leu Lys Gly Glu Ile Ser His Ala Leu Lys Leu Lys
        195                 200                 205

Asp Gly Gly His Tyr Thr Cys Asp Phe Lys Thr Val Tyr Lys Ala Lys
    210                 215                 220

Lys Pro Val Gln Leu Pro Gly Asn His Tyr Val Asp Ser Lys Leu Asp
225                 230                 235                 240

Ile Thr Asn His Asn Glu Asp Tyr Thr Val Val Glu Gln Tyr Glu His
                245                 250                 255

Ala Glu Ala Arg His Ser Gly Ser Gln Val Glu His His His His His
            260                 265                 270

His

<210>  96
<211>  14
<212>  PRT
<213>  Artificial

<220>
<223>  Synthetic

<400>  96

Asn Tyr Thr Asn Asn Tyr Ser Asn Ile Ser Asn Asn Tyr Ser
1               5                   10

## Description

### Technical Field

The present invention relates to a method of ligating a signal sequence and a glycosylation sequence to a polynucleotide encoding a protein to be expressed in a eukaryotic cell and allowing the protein to express in a eukaryotic or animal cell and releasing the expressed protein to the outside of the cell. The present invention also relates to a polynucleotide, a vector, and a host cell used for such method.

### Background Art

A protein translated by a gene plays a key role in body function. As the gene recombination techniques have made progress, methods for target gene expression and methods for purification thereof have been developed.

It is preferable that mammalian proteins be expressed in cells in which such proteins are naturally expressed. When such cells cannot be used, however, use of cells that are phylogenetically close to the original cells is preferable from the viewpoint of protein folding or other factors. Thus, use of eukaryotic cells is preferable for the production of mammalian recombinant proteins. However, many recombinant proteins are produced and accumulated in cells. Since production of such proteins requires complicated processes of purification, protein production would be time-consuming, and mass production thereof was difficult.

Accordingly, a variety of techniques for recombinant protein production involving the use of yeast; for example, a method in which a signal sequence is ligated to a polynucleotide sequence encoding a recombinant protein to be expressed in a cell and the expressed recombinant protein is released to the outside of the cell and a variety of vectors used therefor, have been developed (see U.S. Patent No. 5,010,003, U.S. Patent No. 4,588,684, and WO 00/09718).

In accordance with conventional techniques, however, the efficiency for releasing, for example, a protein with a relatively high molecular weight to the outside of the cell was not sufficiently high, and development of a system that can more efficiently release a recombinant protein to the outside of the cell has been awaited.

### Disclosure of the Invention

The present invention is intended to provide a method of efficiently utilizing a recombinant protein by allowing the recombinant protein to express in a eukaryotic host cell and releasing the expressed recombinant protein to the outside of the cell.

The present inventors have conducted concentrated studies regarding a method in which a recombinant protein is expressed in a host cell and released to the outside of the cell, thus allowing more efficient production of a protein than conventional techniques. The present inventors discovered that, when a recombinant protein is expressed in a host cell, sugar chain modification, such as expression of a polynucleotide encoding a transitional endoplasmic reticulum signal peptide via fusion to an upstream region of a polynucleotide encoding the target recombinant protein to be produced and ligation of a polynucleotide encoding a glycosylation sequence, would result in efficient release (i.e., secretion) of the target protein to the outside of the cell.

The present inventors discovered that use of a signal sequence exemplified as a signal sequence of a transitional endoplasmic reticulum (i.e., interleukin 4, interleukin 5, interleukin 6, interleukin 12, interleukin 13, or interleukin 31) and a glycosylation sequence of any such interleukin enables efficient release of a target protein to the outside of the host cell. This has led to the completion of the present invention.

Further, the present inventors discovered that release of the protein expressed via fusion of the transitional endoplasmic reticulum signal sequence to the artificially designed glycosylation sequence to the outside of the cell would not be influenced by the type of transitional endoplasmic reticulum signal sequence, would be significantly influenced by the presence of sugar chain modification, and would not be significantly influenced by the constitution of the peptide sequence to be released.

It was verified in the present invention that, when the target protein expressed as a fusion protein in a downstream region of the fusion protein of the transitional endoplasmic reticulum signal sequence and the glycosylation sequence is expressed in an adhesive cell (i.e., Cos-1 fibroblast) and in a suspension cell with the aid of an epidermic cell (i.e., the Freestyle 293-F cell), a sugar chain was added upon insertion of the glycosylation sequence, and the target protein was efficiently released to the outside of the cell. Such efficient protein release was observed in the fibroblast, the epidermic cell, the suspension cell, and the adhesive cell. This indicates that protein release is not influenced by cell type.

Mutant analysis demonstrated that a protein would not be efficiently released to the outside of the cell without sugar chain addition. Further, sugar-chain-degrading enzyme-based analysis demonstrated that a protein into which a glycosylation sequence had been introduced would experience modification of N-type glycosylation.

Since protein release was also accelerated by the artificially designed glycosylation sequence, the importance of the presence of the N-type sugar chain was approved, and sugar chain modification was found to be more important than the primary structure.

Two types of fluorescent proteins, murine interleukin 33 (i.e., cytokine, which would not be released in full length), and human p53 protein (i.e., nucleoprotein) were efficiently released in the culture supernatant according to the method of the present invention. This demonstrates the effects of the present invention.

As a result of comparison of transitional endoplasmic reticulum signal sequences, some differences were observed in protein release, although there were no significant differences. This indicates that the influence of the type of transitional endoplasmic reticulum signal sequence was smaller than that of glycosylation. It also indicates that what is important is the presence of the transitional endoplasmic reticulum signal sequence, with the type of such signal sequence not being significant. With the use of the signal sequence of murine interleukin 33, however, substantially no extracellular protein release occurred. Thus, the fact that the type of such signal sequence is not significant does not indicate the lack of necessity of selection of a signal sequence.

Since the human p53 protein, which is a relatively large human tumor-suppressor gene product, was released with activity, the technique of the present invention was found to be effective for the production of active proteins.

Also, a protein was released into a low-protein medium, and it facilitated protein purification. Thus, the technique of the present invention was found to be effective for protein production, including purification.

Specifically, the present invention is as follows.
[1] A polynucleotide used for producing a recombinant protein in a eukaryotic host cell comprising a polynucleotide encoding a transitional endoplasmic reticulum signal sequence and, in a downstream region thereof, a polynucleotide encoding a fusion protein of a protein with an N-type glycosylation sequence consisting of the sequence represented by: Asn-X-(Thr/Ser) (wherein X is an amino acid other than proline), the polynucleotide being used for releasing the protein to the outside of the eukaryotic host cell.
[2] A polynucleotide used for producing a recombinant protein in a eukaryotic host cell comprising a polynucleotide encoding a transitional endoplasmic reticulum signal sequence and, in a downstream region thereof, a polynucleotide encoding a fusion protein of a protein with an O-type glycosylation sequence, the polynucleotide being used for releasing the protein to the outside of the eukaryotic host cell.
[3] The polynucleotide according to [1] or [2], wherein the transitional endoplasmic reticulum signal sequence is selected from the group consisting of a signal sequence of murine interleukin 4 (SEQ ID NO: 1), a signal sequence of murine interleukin 5 (SEQ ID NO: 3), a signal sequence of murine interleukin 6 (SEQ ID NO: 5), a signal sequence of murine interleukin 12 (SEQ ID NO: 7), a signal sequence of murine interleukin 13 (SEQ ID NO: 9), a signal sequence of murine interleukin 31 (SEQ ID NO: 11), a signal sequence of human interleukin 13 (SEQ ID NO: 13), and a signal sequence of human interleukin 31 (SEQ ID NO: 15).
[4] An expression vector comprising the polynucleotide according to any of [1] to [3], which expresses a recombinant protein and releases the expressed protein to the outside of the eukaryotic host cell.
[5] A eukaryotic host cell comprising the expression vector according to [4].
[6] An expression vector used for producing a recombinant protein in a eukaryotic host cell and for releasing the target protein to the outside of the eukaryotic host cell, which comprises a polynucleotide encoding a transitional endoplasmic reticulum signal sequence, in a downstream region thereof, a polynucleotide encoding an N-type glycosylation sequence consisting of the sequence represented by: Asn-X-(Thr/Ser) (wherein X is an amino acid other than proline), and a multicloning site capable of introducing a foreign gene encoding a target protein to be expressed into a downstream region of the polynucleotide encoding a transitional endoplasmic reticulum signal sequence and an upstream or downstream region of the polynucleotide encoding an N-type glycosylation sequence.
[7] An expression vector used for producing a recombinant protein in a eukaryotic host cell and for releasing the target protein to the outside of the host cell, which comprises a polynucleotide encoding a transitional endoplasmic reticulum signal sequence, in a downstream region thereof, a polynucleotide encoding an O-type glycosylation sequence, and a multicloning site capable of introducing a foreign gene encoding a target protein to be expressed into a downstream region of the polynucleotide encoding the transitional endoplasmic reticulum signal sequence and an upstream or downstream region of the polynucleotide encoding an O-type glycosylation sequence.
[8] A method for producing a protein comprising introducing the polynucleotide according to any of [1] to [3] into a eukaryotic host cell, culturing the eukaryotic host cell, expressing the protein encoded by the polynucleotide, releasing the expressed protein to the outside of the cell, and recovering a target protein from a cell culture supernatant.
[9] A method for producing a protein comprising introducing the expression vector according to [6] or [7] into a eukaryotic host cell, culturing the eukaryotic host cell, expressing the protein encoded by the polynucleotide, releasing the expressed protein to the outside of the cell, and recovering a target protein from a cell culture supernatant.
[10] A protein produced by the method according to [8] or [9].
[11] The protein according to [10], which is subjected to sugar chain modification via addition of an N-type glycosylation sequence consisting of the sequence represented by: Asn-X-(Thr/Ser) (wherein X is an amino acid other than proline) thereto and binding of an N-type sugar chain to the N-type glycosylation sequence.
[12] The protein according to [11], which is subjected to sugar chain modification via addition of an N-type glycosylation sequence consisting of the sequence represented by: Asn-X-(Thr/Ser) (wherein X is an amino acid other than proline) thereto, the protein not naturally undergoing sugar chain modification, and binding of an N-type sugar chain to the N-type glycosylation sequence.
[13] The protein according to [10], which is subjected to sugar chain modification via addition of an O-type glycosylation sequence thereto and binding of an O-type sugar chain to the O-type glycosylation sequence.
[14] The protein according to [13], which is subjected to sugar chain modification via addition of an O-type glycosylation sequence thereto, the protein not naturally undergoing sugar chain modification, and binding of an O-type sugar chain to the O-type glycosylation sequence.
[15] A method for producing an antibody via DNA immunization comprising introducing the polynucleotide according to any of [1] to [3] into a non-human animal, expressing a protein encoded by the polynucleotide in the animal body, and producing an antibody against the protein.
[16] A polynucleotide used for producing a recombinant protein in a host cell and releasing the target protein to the outside of the host cell via sugar chain modification, the host cell comprising a polynucleotide encoding a glycosylation sequence consisting of a transitional endoplasmic reticulum signal sequence and the sequence represented by: Asn-X-(Thr/Ser) (wherein X is an amino acid other than proline) and a polynucleotide encoding a target protein, which is not efficiently released to the outside of the host cell even upon fusion with a transitional endoplasmic reticulum signal sequence.

This description includes part or all of the contents as disclosed in the description and/or drawings of Japanese Patent Application No. 2008-149275, which is a priority document of the present application.

### Brief Description of the Drawings

Fig. 1 is a photograph showing the results of Western blot analysis of a protein expressed in the Freestyle-293F cell with the use of an expression vector carrying polynucleotides encoding the amino acid sequences as shown in SEQ ID NOs: 45, 46, 47, 48, 49, and 50.
Fig. 2 schematically shows the locations of the signal sequence and the glycosylation sequence of mutants comprising the amino acid sequences as shown in SEQ ID NOs: 44, 60, 61, 62, 63, 50, and 64.
Fig. 3 is a photograph showing the results of Western blot analysis of a protein expressed in the Freestyle-293F cell with the use of an expression vector carrying polynucleotides encoding the amino acid sequences as shown in SEQ ID NOs: 60, 61, 62, and 63.
Fig. 4 is a photograph showing the results of Western blot analysis of a protein expressed in the Cos-1 cell with the use of an expression vector carrying polynucleotides encoding the amino acid sequences as shown in SEQ ID NOs: 44, 60, 61, 62, and 63.
Fig. 5 is a photograph showing the results of Western blot analysis of a protein expressed in the Freestyle-293F cell with the use of an expression vector carrying polynucleotides encoding the amino acid sequences as shown in SEQ ID NOs: 62 and 63.
Fig. 6 is a photograph showing the results of Western blot analysis of a protein expressed in the Freestyle-293F cell with the use of an expression vector carrying a polynucleotide encoding the amino acid sequence as shown in SEQ ID NO: 64.
Fig. 7 is a photograph showing the results of Western blot analysis of a protein expressed in the Freestyle-293F cell with the use of an expression vector carrying polynucleotides encoding the amino acid sequences as shown in SEQ ID NOs: 67, 68, and 69.
Fig. 8 is a diagram showing the sequence-selective DNA-binding ability of the p53 protein expressed in the Freestyle-293F cell with the use of an expression vector carrying polynucleotides encoding the amino acid sequences as shown in SEQ ID NOs: 67 and 69.
Fig. 9 is a photograph showing the results of purification attained by expressing a protein in the Freestyle-293F cell with the use of an expression vector carrying a polynucleotide encoding the amino acid sequence as shown in SEQ ID NO: 69 and purifying the supernatant with the use of a nickel-chelating column.
Fig. 10 is a photograph showing the results of Western blot analysis of a protein expressed in the Freestyle-293F cell with the use of an expression vector carrying polynucleotides encoding the amino acid sequences as shown in SEQ ID NOs: 73 and 74.
Fig. 11 is a photograph showing the results of Western blot analysis of a protein expressed in the Freestyle-293F cell with the use of an expression vector carrying polynucleotides encoding the amino acid sequences as shown in SEQ ID NOs: 84, 90, 91, 92, 93, 94, and 95.

### Best Modes for Carrying out the Invention

Hereafter, the present invention is described in detail.

The signal sequence of the present invention comprises 15 to 30 amino acids that bind to the signal recognition particle (SRP) proteins (i.e., GTP-binding regulatory proteins existing in the endoplasmic reticulum), and such signal sequence is the transitional endoplasmic reticulum having a hydrophobic core mainly comprising 5 to 10 continuous hydrophobic amino acids at the N-terminus.

Examples of hydrophobic amino acids include glycine, alanine, valine, leucine, isoleucine, proline, methionine, phenylalanine, tyrosine, and tryptophan. Hydrophobic amino acids in a signal sequence may be the same or different amino acids.

At the time of protein translation in the ribosome, a signal sequence is first synthesized, and the signal sequence is recognized by SRP. Thereafter, translation is temporarily discontinued, SRP binds to the ribosome, and the resulting complex binds to an SRP receptor on the endoplasmic reticulum membrane. Once the signal sequence is dissociated from SRP and transferred into the endoplasmic reticulum through pores on the endoplasmic reticulum membrane, translation is restarted, and a protein enters into the endoplasmic reticulum. The signal sequence is cleaved by a peptidase in the endoplasmic reticulum, the protein is transported to the Golgi apparatus, and the protein is then released to the outside of the cell.

The signal sequence of the present invention may be composed of any sequence, provided that such sequence has activity as a signal of the transitional endoplasmic reticulum. Such signal sequence is preferably a peptide sequence, which is recognized and cleaved by a signal peptide recognition mechanism. Examples of such signal sequences include those of interleukin 4, interleukin 5, interleukin 6, interleukin 12, interleukin 13, and interleukin 31. The nucleotide sequences encoding signal sequences of murine interleukin 4, murine interleukin 5, murine interleukin 6, murine interleukin 12, murine interleukin 13, and murine interleukin 31 are shown in SEQ ID NOs: 1, 3, 5, 7, 9, and 11, respectively, and the amino acid sequences of such signal sequences are shown in SEQ ID NOs: 2, 4, 6, 8, 10, and 12, respectively. The nucleotide sequences encoding signal sequences of human interleukin 13 and human interleukin 31 are shown in SEQ ID NOs: 13 and 15, and the amino acid sequences of such signal sequences are shown in SEQ ID NOs: 14 and 16.

When a signal sequence of a protein having a signal sequence is used, a polynucleotide encoding a signal sequence may be selectively ligated to an upstream region of the target protein, and a polynucleotide encoding such protein having a signal sequence and encoding a continuous fragment containing a signal sequence may be ligated to an upstream region of the target protein.

An N-type glycosylation sequence (i.e., the N-type sugar chain modification sequence) is an N-type glycosylation sequence represented by the amino acid sequence: Asn-X-(Thr/Ser). In this formula, X represents any amino acid other than proline, and Thr/Ser represents Thr or Ser. An N-linked sugar chain binds to Asn in Asn-X-(Thr/Ser)(NXT/S) in an N-linked form. Specific examples of such sequences include NYS, NYT, NAS, NAT, NTS, and NTT. In the presence of a glycosylation sequence, a sugar chain is added to a protein in the Golgi apparatus, following migration to the endoplasmic reticulum, and the protein is released to the outside of the cell without regulation.

In addition to an N-type glycosylation sequence, an O-type glycosylation sequence can be used in the present invention. In an expression experiment upon fusion with an EGFP protein, a protein released to the outside of the cell exhibited an increase in a molecular weight via electrophoresis, which indicates O-type sugar chain modification. This demonstrates that O-type sugar chain modification also has the activity of enhancing the efficiency for extracellular release of proteins, as well as N-type sugar chain modification.

In the present invention, target proteins that are produced as recombinant proteins and released to the outside of the cells are not limited, and any proteins can be produced by the method of the present invention.

A preferable example is a protein that is not released to the outside of the cell. An example of such protein is a protein that would not experience sugar chain modification in nature. Further examples include proteins that are not released to the outside of the cell, such as a cytoplasmic protein and a nucleoprotein. A still further example is an extracellular secretion protein that is released without regulation upon conversion of a signal sequence and addition of a sugar chain sequence. A specific example of such protein is murine IL-33.

When a polynucleotide encoding a glycosylation sequence is ligated to a polynucleotide encoding a target protein to be released to the outside of the cell and the resultant is used, a polynucleotide encoding a glycosylation sequence may be ligated to an upstream region (5'-side) or a downstream region (3'-side) of a polynucleotide encoding a target protein. In such a case, the resulting sequence comprises a polynucleotide encoding a transitional endoplasmic reticulum signal sequence and, in a downstream region thereof, a polynucleotide encoding a fusion protein of a protein and an O-type glycosylation sequence added thereto.

SEQ ID NO: 17 shows the polynucleotide sequence (GSS-artificial) comprising an N-type sugar chain modification sequence ligated to a downstream region (3'-side) of a polynucleotide encoding a signal sequence of murine interleukin 31. SEQ ID NO: 18 shows the amino acid sequence (the GSS amino acid) encoded by such polynucleotide. In the amino acid sequence as shown in SEQ ID NO: 18, a glycosylation sequence is located in a downstream region of the transitional endoplasmic reticulum signal sequence. This glycosylation sequence is an artificially designed sequence, which does not exist in nature. N-type sugar chains can be added to four amino acid regions. Upon fusion of a fluorescent protein to a downstream region of such sequence, a sugar chain was added, and the protein was released to the outside of the cell. Accordingly, the glycosylation sequence is not limited to glycosylation sequences existing in nature.

Also, a polynucleotide encoding a protein having a transitional endoplasmic reticulum signal sequence and a glycosylation sequence in nature, including a polynucleotide encoding a signal sequence contained in the gene of the protein, may be used. In such a case, a continuous sequence of a polynucleotide spanning from the signal sequence of the gene of the protein to at least the glycosylation sequence of an open reading frame (ORF) encoding the protein may be ligated to an upstream region (5'-side) of a polynucleotide encoding a target protein to be produced. A polynucleotide encoding a full-length sequence containing a signal sequence of a naturally-occurring protein may be ligated to a polynucleotide encoding a target protein.

Examples of polynucleotides encoding naturally-occurring proteins comprising signal sequences and glycosylation sequences include polynucleotides encoding interleukin 13 and interleukin 31. Interleukin 13 and interleukin 31 may be derived from any animal species without limitation, and human-derived and mouse-derived interleukins can be preferably used.

Examples include a polynucleotide encoding murine interleukin 31 comprising a signal sequence as shown in SEQ ID NO: 19 and a polynucleotide encoding human interleukin 31 comprising a signal sequence as shown in SEQ ID NO: 21. Amino acid sequences encoded by such polynucleotides are shown in SEQ ID NO: 20 and SEQ ID NO: 22, respectively.

A polynucleotide that can hybridize under stringent conditions to a polynucleotide consisting of a nucleotide sequence consisting of a sequence complementary to the aforementioned nucleotide sequence, which consists of a nucleotide sequence encoding a protein having activity of the protein encoded by the polynucleotide consisting of the nucleotide sequence as shown in SEQ ID NO: 19 or 21, may also be used. The term "stringent conditions" used herein refers to, for example, 1x SSC and 0.1% SDS at 37°C, the term "more stringent conditions" refers to, for example, 0.5x SSC and 0.1% SDS at 42°C, and the term "further stringent conditions" refers to, for example, 0.2x SSC and 0.1% SDS at 65°C. As the degree of stringency for hybridization is increased, detection and isolation of DNA with higher homology can be expected. It should be noted that the aforementioned combinations of SSC, SDS, and temperature conditions are exemplary, and a person skilled in the art would be able to realize the degree of stringency equivalent to the above by adequately combining the above-mentioned and other factors that determine the degree of stringency for hybridization (e.g., polynucleotide concentration, polynucleotide length, and the duration of hybridization). In addition, a polynucleotide consisting of a nucleotide sequence having 80% or higher, preferably about 90% or higher, and more preferably about 95% or higher identity with the nucleotide sequence as shown in SEQ ID NO: 19 or 21, which is determined with the use of default parameters (default configurations) of a homology search program, such as BLAST, may be used.

When a target protein to be expressed naturally has a glycosylation sequence as exemplified by murine interleukin 33, the glycosylation sequence may be fused with a transitional endoplasmic reticulum signal sequence, so that a sugar chain is added to the glycosylation sequence, and extracellular protein release is accelerated. Under such circumstances, such glycosylation sequence existing in nature can be used. Thus, it is not always necessary to ligate a polynucleotide encoding a glycosylation sequence to an upstream or downstream region of a polynucleotide encoding a target protein. The nucleotide sequence of murine interleukin 33 is shown in SEQ ID NO: 23, and the amino acid sequence thereof is shown in SEQ ID NO: 24. In this case, such polynucleotide is within the scope of "the polynucleotide encoding a transitional endoplasmic reticulum signal sequence and, in a downstream region thereof, a polynucleotide encoding a fusion protein of a protein with an O-type glycosylation sequence or an N-type glycosylation sequence consisting of the sequence represented by: Asp-X-(Thr/Ser) (wherein X is an amino acid other than proline)" of the present invention. Also, a polynucleotide capable of hybridizing under stringent conditions to a polynucleotide consisting of a nucleotide sequence complementary to the nucleotide sequence as shown in SEQ ID NO: 23 and consisting of a nucleotide sequence encoding a protein having activity of the protein encoded by the polynucleotide consisting of nucleotide sequence as shown in SEQ ID NO: 23 may be used. In addition, a polynucleotide consisting of a nucleotide sequence having 80% or higher, preferably about 90% or higher, and more preferably about 95% or higher identity with the nucleotide sequence as shown in SEQ ID NO: 23, which is determined with the use of default parameters (default configurations) of a homology search program, such as BLAST, may be used.

The present invention includes a polynucleotide used for expressing a target protein in a host cell and releasing (secreting) the target protein to the outside of the host cell. Example of such polynucleotide include a polynucleotide encoding a transitional endoplasmic reticulum signal sequence, a polynucleotide encoding an O-type glycosylation sequence or an N-type glycosylation sequence consisting of the sequence represented by: Asp-X-(Thr/Ser) (wherein X is an amino acid other than proline), and a polynucleotide encoding the target protein. Such polynucleotide comprises, in a downstream region of a polynucleotide encoding a transitional endoplasmic reticulum signal, a polynucleotide encoding a glycosylation sequence and a polynucleotide encoding a target protein. A polynucleotide encoding a target protein and a polynucleotide encoding a glycosylation sequence may be a polynucleotide encoding a fusion protein of a target protein and a glycosylation sequence added thereto. A polynucleotide encoding such fusion protein is located in a downstream region of a polynucleotide encoding a transitional endoplasmic reticulum signal sequence. A plurality of glycosylation sequences may be ligated. Specifically, a polynucleotide used for expressing the protein of the present invention in a eukaryotic cell and releasing the expressed protein to the outside of the cell consisits of, for example, a polynucleotide encoding a transitional endoplasmic reticulum signal, a polynucleotide encoding the sequence represented by: {Asn-X-(Thr/Ser)}_{N} (wherein N is an integer of 1 to 5), and a polynucleotide encoding a target protein. Other nucleotide sequences may be included among a polynucleotide encoding a transitional endoplasmic reticulum signal, a polynucleotide encoding a glycosylation sequence, and a polynucleotide encoding a target protein.

It was actually demonstrated by the experiment involving the use of a red fluorescent protein (Dsred) and a green fluorescent protein (EGFP) that expression of proteins in an artificially designed N-type glycosylation sequence consisting of 14 amino acid residues as shown in the NYTNNYSNISNNYS sequence (SEQ ID NO: 96) in a downstream region of the transitional endoplasmic reticulum signal sequence would cause sugar chain addition and extracellular release of proteins produced along therewith would be induced.

A promoter may be operably linked to an upstream region of such polynucleotide. Any promoter may be used in the present invention, provided that such promoter is suitable for a host used for gene expression. When an yeast host is used, for example, PHO5 promoter, PGK promoter, GAP promoter, ADH promoter, or the like is preferable. When an animal host cell is used, examples of promoters include SRα promoter, SV40 promoter, LTR promoter, CMV promoter, and HSV-TK promoter. Also, an inducible promoter that is induced to function upon addition of an agent (i.e., an inducer) or other specific conditions may be used.

In the present invention, the polynucleotide used for expressing the target recombinant protein in a eukaryotic cell and secreting the expressed protein to the outside of the cell may further comprise an enhancer, a splicing signal, a poly A addition site, a selection marker, an SV40 replication origin, and the like that are known in the art.

The present invention also includes an expression cassette used for producing a recombinant protein in a host cell comprising a polynucleotide encoding a transitional endoplasmic reticulum signal sequence, in a downstream region thereof, a polynucleotide encoding a fusion protein of a protein and an O-type glycosylation sequence or an N-type glycosylation sequence consisting of the sequence represented by: Asn-X-(Thr/Ser) (wherein X is an amino acid other than proline) added thereto, a promoter, and the like.

The present invention includes a method for producing a protein comprising introducing a polynucleotide encoding a transitional endoplasmic reticulum signal sequence and, in a downstream region thereof, a polynucleotide encoding a fusion protein of a protein and an N-type glycosylation sequence consisting of the sequence represented by: Asn-X-(Thr/Ser) (wherein X is an amino acid other than proline) added thereto into a eukaryotic host cell, culturing the eukaryotic host cell, expressing a protein encoded by such polynucleotide, releasing the expressed protein to the outside of the cell, and recovering a target protein from a cell culture supernatant.

In the present invention, the polynucleotide used for expressing a target recombinant protein in a eukaryotic cell and releasing the target protein to the outside of the cell may be incorporated into a vector, and the resultant may then be introduced into a eukaryotic host cell.

Examples of eukaryotic cells used in the method of the present invention include yeast, insect, avian, amphibian, reptile, and mammalian cells.

Examples of yeast include *Saccharomyces cerevisiae* AH22, AH22R-, NA87-11A, DKD-5D, and 20B-12, *Schizosaccharomyces pombe* NCYC1913 and NCYC2036, and *Pichia pastoris.*

Examples of insect cells include Mamestra cells, such as Sf21 cells.

Examples of amphibian cells include Xenopus egg cells.

Examples of mammalian cells include: human cells, such as HEK293 cells, FreeStyle 293 cells, and FL cells; monkey cells, such as COS-7 and Vero cells; Chinese hamster cells, such as CHO and the dhfr gene-deficient CHO cells; mouse cells, such as mouse L cells, mouse AtT-20 cells, and mouse myeloma cells; and rat cells, such as rat GH3 cells.

Examples of expression vectors include pKA1, pCDM8, pSVK3, pSVL, pBK-CMV, pBK-RSV, EBV, pRS, and pYE82 vectors. If pIND/V5-His, pFLAG-CMV-2, pEGFP-N1, or pEGFP-C1 vectors are used as expression vectors, target proteins can be expressed in the form of fusion proteins to which a variety of tags, such as His, FLAG. or GFP tags, have been added.

Use of cells that can be cultured at low protein concentrations is particularly preferable since cells can be easily purified from a culture supernatant. An example of cells that can be cultured at low protein concentrations is the FreeStyle 293 cells.

A vector comprises a polynucleotide used for producing a recombinant protein in a host cell comprising a polynucleotide encoding a transitional endoplasmic reticulum signal sequence, a polynucleotide encoding an O-type glycosylation sequence or an N-type glycosylation sequence consisting of the sequence represented by: Asn-X-(Thr/Ser) (wherein X is an amino acid other than proline), and a polynucleotide encoding a target protein. The present invention includes an expression vector used for expressing such recombinant protein and releasing the expressed protein to the outside of the host cell and a host cell into which such vector has been introduced.

As a site to which a target protein is to be ligated, a multicloning site may be incorporated, and a foreign gene encoding a target protein may be incorporated into such multicloning site. In such a case, the expression vector of the present invention is used for producing a recombinant protein in a host cell, which comprises a polynucleotide encoding a transitional endoplasmic reticulum signal sequence, in a downstream region thereof, a polynucleotide encoding an O-type glycosylation sequence or an N-type glycosylation sequence consisting of the sequence represented by: Asn-X-(Thr/Ser) (wherein X is an amino acid other than proline), and a multicloning site into which a foreign gene encoding the target protein can be introduced.

A recombinant vector may be introduced into an yeast cell by any method without particular limitation, provided that DNA can be introduced into an yeast cell. Examples thereof include electroporation (Becker, D. M. et al., Methods, Enzymol., 194: 182, 1990), the spheroplast method (HiNNen, A. et al., Proc. Natl. Acad. Sci., U.S.A., 75; 1929, 1978), and a lithium acetate method (Itoh, H., J. Bacteriol., 153: 163, 1983). A recombinant vector may be introduced into an animal cell via, for example, electroporation, the calcium phosphate method, or lipofection.

According to the present invention, a eukaryotic host cell into which a vector comprising a polynucleotide used for expressing a target recombinant protein in the eukaryotic cell and secreting the target protein to the outside of the cell has been introduced may be cultured to express the target protein, and the expressed target protein can then be released to the outside of the cell (i.e., into a culture supernatant). Culture is carried out in accordance with a conventional technique used for host cell culture.

After the completion of culture, cells are separated from a supernatant in accordance with a conventional technique, and a supernatant is collected. Proteins contained in the thus-obtained culture supernatant or an extract may be purified by adequately combining conventional separation and purification techniques. In comparison with proteins extracted from cells, released proteins contain less impurities or contaminants, and use of a surfactant is not necessary at the time of extraction. In this respect, such method is effective for recovery of active proteins. Examples of such techniques include treatment with the use of a modifier such as urea or a surfactant, ultrasonication, enzyme digestion, salting out or solvent precipitation, dialysis, centrifugation, ultrafiltration, gel filtration, SDS-PAGE, isoelectric focusing, ion exchange chromatography, hydrophobic chromatography, affinity chromatography, and reverse phase chromatography.

A sugar chain can be removed from the produced protein with the use of a sugar-chain-degrading enzyme. However, novel activity may be occasionally imparted to the expressed protein via glycosylation. In such a case, a glycosylation sequence is effective for preparation of a useful sugar chain protein.

The present invention includes a protein produced by the method of the present invention. Such protein is translated and it is then occasionally subjected to various types of modification in a cell. Accordingly, a modified protein is within the scope of the protein of the present invention. Examples of post-translational modification include elimination of N-terminal methionine, N-terminal acetylation, limited degradation by intracellular protease, myristoylation, isoprenylation, and phosphorylation.

The protein expression vector of the present invention comprising a polynucleotide encoding a transitional endoplasmic reticulum signal sequence and, in a downstream region thereof, a polynucleotide encoding a fusion protein of a protein and an O-type glycosylation sequence or an N-type glycosylation sequence consisting of the sequence represented by: Asn-X-(Thr/Ser) (wherein X is an amino acid other than proline) added thereto can be used for DNA immunization. Specifically, a eukaryotic cell expression vector is introduced into the muscle or skin of an animal by means of an injection or gene gun, and the expressed proteins are then released into the blood. This results in immunization, and the blood serum reacting with the target protein can be sampled. Thus, a protein expression vector of interest can be produced. Examples of animals that can be used include mice, rats, rabbits, goats, and chickens. B cells sampled from the spleen of the immunized animal may be fused with myeloma cells to prepare hybridomas, and monoclonal antibodies can then be produced.

Since the introduced proteins are released to the outside of the cell, such proteins can be used for genetic therapy and DNA vaccines. Further, cells that produce extracellular secretion proteins may be established and used for cell therapy. Also, proteins that exist in the cells and are usually recognized as autologous proteins may be released to the outside of the cell to induce immunogenicity, and the resultant may be used for preparing mouse models of autoimmune disease.

### Examples

The present invention is described in greater detail with reference to the examples below, although the technical scope of the present invention is not limited to these examples. Basic procedures regarding DNA recombinations and enzyme reactions were in accordance with the literature, "Molecular Cloning: A Laboratory Manual," Cold Spring Harbor Laboratory, 1989. Restriction enzymes and various modification enzymes available from Invitrogen were used, unless otherwise specified. The compositions of buffers and reaction conditions for enzyme reactions were in accordance with the accompanying instructions.

### Method

### (1) Construction of expression vector

In order to prepare cDNA comprising the Kozak sequence and the EcoRI site at the N terminus and the XhoI site at the C terminus, PCR was carried out using the primer sequences (SEQ ID NO: 25 and SEQ ID NO: 26) and cDNA of the spleen cells of Balb/C mice as the template in accordance with the instructions of the Phusion PCR kit (Finnzyme, Finland). PCR was carried out at 96°C for 2 minutes, 35 cycles of 98°C for 15 seconds, 58°C for 15 seconds, and 72°C for 1 minute, and 72°C for 1 minute. PCR was carried out under such conditions, unless otherwise specified.

The obtained PCR fragment (574 bp) was phosphorylated with the aid of T4 polynucleotide kinase (Invitrogen) in the presence of ATP (Invitrogen), the blunt-ended fragment was cloned into the EcoRV site of the pBluescript 2 SK+ cloning vector, and a clone comprising the sequence as shown in SEQ ID NO: 27 corresponding to the relevant region of the Genbank XM_132344 clone was obtained.

In order to fuse the EGFP protein and a histidine tag to the C terminus of murine IL-31, PCR was carried out using the aforementioned plasmid as a template and primer sequences (SEQ ID NO: 25 and SEQ ID NO: 28), which comprise the Kozak sequence and the EcoRI site at the N terminus and a sequence converting the termination codon at the C terminus into the XhoI site, the resultant was subcloned into the EcoRV site of the pBluescript 2 Sk+ vector, and a clone having full-length cDNA without mutation was obtained (mIL31-fus/pBSK2+).

In order to fuse the EGFP protein and a histidine tag to the C terminus of murine IL-4, 5, 6, 12, and 13, respectively, similarly, PCR was carried out using primer sequences, which comprise the Kozak sequence and the EcoRI site at the N terminus and a sequence converting the termination codon at the C terminus into the BamHI site, and cDNA of the spleen cells of Balb/C mice as the template, the resultants were subcloned into the EcoRV site of pBluescript 2 SK+ as in the case of mIL-31, and a clone having full-length cDNA without mutation was obtained (mIL-x-fus/pBSK2+ (X = 4, 5, 6, 12, and 13)).

Primers as shown in SEQ ID NOs: 29 and 30 were used for murine IL-4, primers as shown in SEQ ID NOs: 31 and 32 were used for murine IL-5, primers as shown in SEQ ID NOs: 33 and 34 were used for murine IL-6, primers as shown in SEQ ID NOs: 35 and 36 were used for murine IL-12, and primers as shown in SEQ ID NOs: 37 and 38 were used for murine IL-13.

In order to conduct an analysis with the use of the enhanced green fluorescent protein (Jelly fish) (EGFP), the multiple cloning site resulting from annealing of primer sequences (SEQ ID NO: 39 and SEQ ID NO: 40) to a site between the HindIII site and the XhoI site of the mammalian expression vector (i.e., pcDNA3.1-MH-A+, Invitrogen) was modified, and the pcDNA3.1-modified+ plasmid in which the positions of HindIII, EcoRI, BamHI, and XhoI had been modified was obtained.

PCR was carried out using a green fluorescent protein (EGFP) expression vector (i.e., pEGFP-C1, Clonetech) as the template and primers as shown in SEQ ID NOs: 41 and 42 to amplify cDNA of EGFP. The obtained fragment was subcloned into the EcoRV site of pBluescript 2 SK+, and clones, which did not experience mutation during PCR and oligo synthesis, were selected. The plasmid was cleaved with the BamHI and SaII restriction enzymes, and the cleaved fragment was subcloned into a site between the BamHI site and the XhoI site of the pcDNA3.1-modified+ plasmid to obtain an EGFP expression vector (EGFP-H/pcDNA3.1).

This expression vector comprises the polynucleotide sequence shown below (SEQ ID NO: 43) in a downstream region of the CMV promoter and expresses the protein as shown in SEQ ID NO: 44 (EGFP-H).

As a result of such modification, the XhoI site migrates to a new location via ligation.

A fragment obtained by cleaving mIL-X-fus/pBSK2+ (X = 4, 5, 6, 12, and 13) with EcoRI and BamHI was inserted into a site between EcoRI and BamHI of the prepared vector (EGFP-H/pcDNA3.1). Thus, a vector that expresses a fusion protein of full-length murine interleukin, EGFP, and a histidine tag was obtained (mIL-X-EGFPH/pcDNA3.1) (X = 4, 5, 6, 12, and 13).

The mIL-4-EGFPH/pcDNA3.1 expression vector expresses the protein as shown in SEQ ID NO: 45 (mIL-4-EGFPH). The mIL-5-EGFPH/pcDNA3.1 expression vector expresses the protein as shown in SEQ ID NO: 46 (mIL-5-EGFPH). The mIL-6-EGFPH/pcDNA3.1 expression vector expresses the protein as shown in SEQ ID NO: 47 (mIL-6-EGFPH. The mIL-12-EGFPH/pcDNA3.1 expression vector expresses the protein as shown in SEQ ID NO: 48 (mIL-12-EGFPH). The mIL-13-EGFPH/pcDNA3.1 expression vector expresses the protein as shown in SEQ ID NO: 49 (mIL-13-EGFPH).

Similarly, mIL31-fus/pBSK2+ was cleaved with EcoRI and XhoI, and the resulting fragment was introduced into a site between EcoRI and XhoI of the prepared vector (EGFP-MH/pcDNA3.1-MH-A+) to obtain a vector that expresses a fusion protein of full-length murine interleukin 31, EGFP, and a histidine tag (mIL-31-EGFPH/pcDNA3.1).

The mIL-31-EGFPH/pcDNA3.1 expression vector expresses the protein as shown in SEQ ID NO: 50 (mIL-31-EGFPH).

In order to determine a region necessary for extracellular secretion of murine interleukin 31, the vector (mIL-31-EGFPH/pcDNA3.1) was subjected to modification. With the use of the mIL-31-EGFPH/pcDNA3.1 template, a primer (SEQ ID NO: 51) having a sequence within the CMV promoter region of the expression vector, and a primer shown below, a partial sequence of mIL31 was amplified via PCR, the resulting fragment was cleaved with EcoRI and BamHI, and the resultant was introduced into a site between EcoRI and BamHI of the EGFPH/pcDNA3.1 vector to prepare mutants.

The correlation between the sequences of the primers used and the resulting mutant expression vectors are as described below SS-EGFPH/pcDNA3.1 was prepared with the use of the primers as shown in SEQ ID NO: 52 and SEQ ID NO: 51, Δ-EGFPH/pcDNA3.1 was prepared with the use of the primers as shown in SEQ ID NO: 53 and SEQ ID NO: 51, GSS-EGFPH/pcDNA3.1 was prepared with the use of the primers as shown in SEQ ID NO: 54 and SEQ ID NO: 51, and GSS(DD)-EGFPH/pcDNA3.1 was prepared with the use of the primers as shown in SEQ ID NO: 55 and SEQ ID NO: 51.

In order to construct signal sequences and artificial sugar chain modification sites, PCR was carried out using the mIL-31-EGFPH/pcDNA3.1 template and primers (SEQ ID NO: 51 and SEQ ID NO: 56), and fragments comprising signal sequences were obtained. Separately, PCR was carried out using the synthetic oligo DNA (SEQ ID NO: 57) template and primers (SEQ ID NO: 58 and SEQ ID NO: 59) to prepare fragments comprising artificial sugar chain modification sites. The fragments were purified, the same amounts thereof were mixed with each other, the resulting fragments were used as templates to conduct PCR with the use of the primers (SEQ ID NO: 51 and SEQ ID NO: 59), the two fragments were ligated to each other via PCR, and the resultant was amplified to prepare a cDNA fragment. After purification, the resultant was cleaved with EcoRI and BamHI, and the cleaved fragment was introduced into a site between EcoRI and BamHI of the EGFPH/pcDNA3.1 vector to prepare the SS-Art-EGFPH/pcDNA3.1 expression vector having a signal sequence and an artificial sugar chain modification site. SS-EGFPH/pcDNA3.1 expresses the protein as shown in SEQ ID NO: 60, Δ-EGFPH/pcDNA3.1 expresses the protein as shown in SEQ ID NO: 61, GSS-EGFPH/pcDNA3.1 expresses the protein as shown in SEQ ID NO: 62, GSS(DD)-EGFPH pcDNA3.1 expresses the protein as shown in SEQ ID NO: 63, and SS-Art-EGFPH/pcDNA3.1 expresses the protein as shown in SEQ ID NO: 64.

PCR was carried out with the use of cDNA of the human peripheral mononuclear cell as a template and primers (SEQ ID NO: 65 and SEQ ID NO: 66) to amplify cDNA encoding the human p53 protein comprising the XhoI site at the C terminus. The resulting fragment was phosphorylated, blunt-ended, and cloned into the EcoRV site of pBluescript 2 SK+. Clones free of PCR-induced mutation or mutation during primer synthesis were selected, cleaved with BamHI and XhoI, and subcloned into a site between BamHI and XhoI of pcDNA3.1-myc-His-A+ to obtain the p53 expression vector (p53H/pcDNA3.1). The p53H/pcDNA3.1 vector expresses the protein as shown in SEQ ID NO: 67 (p53 protein).

The h-p53-his/pcDNA3.1-MH-A+ vector was cleaved with BamHI and XbaI, the resulting fragment was introduced into a site between the BamHI site and the XbaI site of SS-EGFPH/pcDNA3.1 or GSS-EGFPH/pcDNA3.1, SS-p53H/pcDNA3.1 was prepared from SS-EGFPH/pcDNA3.1, and GSS-p53H/pcDNA3.1 was prepared from GSS-EGFPH/pcDNA3.1. SS-p53H/pcDNA3.1 expresses the protein as shown in SEQ ID NO: 68 (ss-p53H protein) and GSS-p53H/pcDNA3.1 expresses the protein as shown in SEQ ID NO: 69 (GSS-p53H protein).

### Murine interleukin 33 expression vector

PCR was carried out using mouse spleen cDNA as a template and primers (SEQ ID NOs: 70 and 71) to amplify cDNA encoding murine interleukin 33 (mIL-33) comprising the XhoI site at the C terminus. The resulting fragment was phosphorylated, blunt-ended, and cloned into the EcoRV site of pBluescript 2 SK+. Clones free of PCR-induced mutation or mutation during primer synthesis were selected, cleaved with BamHI and XhoI, and subcloned into a site between BamHI and XhoI of pcDNA3.1-myc-His-A+ to obtain the mIL-33 expression vector (mIL-33H/pcDNA3.1).

PCR was carried out using this vector as a template and primers (SEQ ID NOs: 71 and 72) to amplify a cDNA fragment encoding an mIL-33 mature region. The resulting fragment was phosphorylated, blunt-ended, and cloned into the EcoRV site of pBluescript 2 SK+. Clones free of PCR-induced mutation or mutation during primer synthesis were selected, cleaved with BamHI and XhoI, and subcloned into a site between BamHI and XhoI of SS-p53H/pcDNA3.1 to obtain an expression vector comprising mature mIL-33 in a downstream region of the mIL31 signal sequence (SS-mIL-33H/pcDNA3.1).

mIL-33H/pcDNA3.1 expresses the protein as shown in SEQ ID NO: 73 (mIL-33 protein) and SS-mIL-33H/pcDNA3.1 expresses the protein as shown in SEQ ID NO: 74 (SS-mIL-33H protein).

### Cell culture

HEK-293 and Cos-1 cells were cultured with the use of Dulbecco's modified Eagle's medium (DMEM) (Gibco-Invitrogen), 10% fetal bovine serum (Gibco-Invitrogen) (Hyclone, Logan, UT, U.S.A.), and penicillin/streptomycin medium (Gibco-Invitrogen) at 37°C in the presence of 5% carbon dioxide.

The FreeStyle 293-T (FS-293T) cells (Invitrogen) were subjected to shake culture with the use of a rotary shaker in FreeStyle medium (Invitrogen) at 37°C in the presence of 8% carbon dioxide in accordance with the instructions provided by Invitrogen.

Expression plasmids were transformed into DH5α-FT and purified using the NucleoBond Xtra Midi plasmid purification kit (Macherey-Nagel, Duren, Germany) in accordance with the instructions thereof HEK-293 and Cos-1 cells were transfected with the use of Lipofectamine 2000 (Invitrogen) and FS293-T cells were transfected with the use of 293fectin (Invitrogen) in accordance with the relevant instructions. When the blood serum is used, the low immunoglobulin fetal bovine serum (Invitrogen) was used instead in order to prevent nonspecific detection of immunoglobulin via Western blotting.

The cell culture supernatant was removed 3 days after transfection, centrifuged at 2,000 g for 5 minutes, and designated as the cell supernatant. After the supernatant was removed, HEK-293 and Cos-1 cells were washed twice with PBS in an amount the same as that of the medium, the cells were lysed with 0.5% SDS-containing PBS in an amount the same as that of the medium, and the resultant was subjected to superheating at 100°C for 5 minutes. When FreeStyle-293T was used, cells were washed with PBS buffer via centrifugation, the cells were lysed with 0.5% SDS-containing PBS in an amount the same as that of the medium, and the resultant was subjected to superheating at 100°C for 5 minutes.

### (PBS: 137 mM NaCl, 8.1 mM Na₂HPO₄, 2.68 mM KCI, and 1.47 mM KH₂PO₄, pH 7.4)

### Western blotting

The cell supernatant and the cell fraction extract were subjected to Western blotting with the use of the Hybond-P (PVDF) membrane (Amersham) in accordance with the instructions of the Can Get Signal kit (Toyobo Co., Ltd., Toyama, Japan). The anti-His-tag antibody (PM002, MBL, Japan) was used as the primary antibody, the anti-rabbit-IgG-HRP conjugate antibody (Cat. NA934V, GE Healthcare, U.K.) was used as the secondary antibody, and detection was carried out via exposure to Hyperfilm-ECL (Amersham) in accordance with the instructions of the ECL plus detection kit (Amersham, Oakville, Ontario, Canada).

The obtained image was converted into an electronic image with the use of an image scanner (Chem Doc XRS, Bio-Rad), and densitometry was carried out using Quantity One software included therein.

### Preparation of Art-DsredH/pcDNA3.1

PCR was carried out using a red fluorescent protein expression vector (pDsRed-Monomer-C1, Clontech) as a template and primers (SEQ ID NO: 75 and SEQ ID NO: 76). cDNA encoding a fluorescent protein was amplified, electrophoresed, and purified. Thus, cDNA encoding the nucleotide sequence as shown in SEQ ID NO: 77 comprising 684 nucleotides was obtained.

The primer as shown in SEQ ID NO: 78 was phosphorylated, and the resultant was mixed with the same amount of the primer as shown in SEQ ID NO: 79, followed by annealing and blunt-ending. Thus, cDNA as shown in SEQ ID NO: 80 was obtained. cDNA as shown in SEQ ID NO: 74 was ligated to cDNA as shown in SEQ ID NO: 80, the resultant was purified, and PCR was carried out using the purified cDNA as a template and the phosphorylated primers as shown in SEQ ID NO: 79 and SEQ ID NO: 74 to obtain cDNA as shown in SEQ ID NO: 81 comprising 747 nucleotides. Such cDNA was subcloned into the EcoRV site of pBluescript 2 SK+, and a plasmid containing an artificial glycosylation site free of mutation during primer synthesis or PCR and cDNA encoding a red fluorescent protein was obtained. The pcDNA3.1-MH-A+ vector (Invitrogen) was cleaved with EcoRV and XbaI, synthetic DNAs as shown in SEQ ID NOs: 82 and 83 were annealed thereto, a plasmid resulting from subcloning of the annealed product therein was cleaved with XhoI and EcoRI, a plasmid having the sequence as shown in SEQ ID NO: 81 was cleaved with EcoRI and SalI, and the purified cDNA comprising 747 nucleotides was subcloned to obtain a vector that expresses a red fluorescent protein containing the glycosylation sequence (i.e., Art-DsredH/pcDNA3.1).

Art-DsredH/pcDNA3.1 expresses the mIL-33 mature region as shown in SEQ ID NO: 84 (Art-DsredH protein).

### Isolation of signal sequence of murine interleukin (IL-4/5/6/12/13)

In order to isolate a signal sequence of murine IL-4, PCR was carried out using the mIL-4-EGFPH/pcDNA3.1 plasmid as a template and primers (SEQ ID NO: 51 and SEQ ID NO: 85), the resultant was purified and then cleaved with HindIII and BamHI, the cleaved fragment was subcloned into a site between HindIII and BamHI of the Art-DsredH/pcDNA3.1 plasmid, and a clone having a structure of interest was selected to obtain a vector expressing a fusion protein of a signal sequence of murine IL-4, an artificial glycosylation sequence, a red fluorescent protein, and a histidine tag (SS(mIL4)-Art-DsredH/pcDNA3.1)

In order to isolate a signal sequence of murine IL-5, PCR was carried out using the mIL-5-EGFPH/pcDNA3.1 plasmid as a template and primers (SEQ ID NO: 51 and SEQ ID NO: 86), the resultant was purified and then cleaved with HindIII and BamHI, the cleaved fragment was subcloned into a site between HindIII and BamHI of the Art-DsredH/pcDNA3.1 plasmid, and a clone having a structure of interest was selected to obtain a vector expressing a fusion protein of a signal sequence of murine IL-5, an artificial glycosylation sequence, a red fluorescent protein, and a histidine tag (SS(mIL5)-Art-DsredH/pcDNA3.1).

In order to isolate a signal sequence of murine IL-6, PCR was carried out using the mIL-6-EGFPH/pcDNA3.1 plasmid as a template and primers (SEQ ID NO: 51 and SEQ ID NO: 87), the resultant was purified and then cleaved with HindIII and BamHI, the cleaved fragment was subcloned into a site between HindIII and BamHI of the Art-DsredH/pcDNA3.1 plasmid, and a clone having a structure of interest was selected to obtain a vector expressing a fusion protein of a signal sequence of murine IL-6, an artificial glycosylation sequence, a red fluorescent protein, and a histidine tag (SS(mIL6)-Art-DsredH/pcDNA3.1).

In order to isolate a signal sequence of murine IL-12, PCR was carried out using the mIL-12-EGFPH/pcDNA3.1 plasmid as a template and primers (SEQ ID NO: 51 and SEQ ID NO: 88), the resultant was purified and then cleaved with HindIII and BamHI, the cleaved fragment was subcloned into a site between HindIII and BamHI of the Art-DsredH/pcDNA3.1 plasmid, and a clone having a structure of interest was selected to obtain a vector expressing a fusion protein of a signal sequence of murine IL-12, an artificial glycosylation sequence, a red fluorescent protein, and a histidine tag (SS(mIL12)-Art-DsredH/pcDNA3.1).

In order to isolate a signal sequence of murine IL-13, PCR was carried out using the mIL-5-EGFPH/pcDNA3.1 plasmid as a template and primers (SEQ ID NO: 51 and SEQ ID NO: 89), the resultant was purified and then cleaved with HindIII and BamHI, the cleaved fragment was subcloned into a site between HindIII and BamHI of the Art-DsredH/pcDNA3.1 plasmid, and a clone having a structure of interest was selected to obtain a vector expressing a fusion protein of a signal sequence of murine IL-13, an artificial glycosylation sequence, a red fluorescent protein, and a histidine tag (SS(mILl3)-Art-DsredH/pcDNA3.1).

An expression vector containing a signal peptide of murine IL-31 (i.e., SS-EGFP-MH/pcDNA3.1-MH-A+) was cleaved with HindIII and BamHI, cDNA containing a signal peptide of murine IL-31 was purified via electrophoresis, and the resultant was subcloned into a site between HindIII and BamHI of the Art-DsredH/pcDNA3.1 plasmid to obtain a vector expressing a fusion protein of a signal sequence of murine IL-31, an artificial glycosylation sequence, a red fluorescent protein, and a histidine tag (SS(mIL3 1)-Art-DsredH/pcDNA3.1).

The thus-prepared expression vectors express proteins with the aid of the CMV promoter; i.e., SS(mIL4)-Art-DsredH/pcDNA3.1 expresses the protein as shown in SEQ ID NO: 90, SS(mIL5)-Art-DsredH/pcDNA3.1 expresses the protein as shown in SEQ ID NO: 91, SS(mIL6)-Art-DsredH/pcDNA3.1 expresses the protein as shown in SEQ ID NO: 92, SS(mIL12)-Art-DsredH/pcDNA3.1 expresses the protein as shown in SEQ ID NO: 93, SS(mIL13)-Art-DsredH/pcDNA3.1 expresses the protein as shown in SEQ ID NO: 94, and SS(mIL3 1)-Art-DsredH/pcDNA3.1 expresses the protein as shown in SEQ ID NO: 95.

### Expression of DsRed protein

The plasmids; Art-DsredH/pcDNA3.1 and SS (mIL-X)-Art-DsredH/pcDNA3.1 (X = 4, 5, 6, 12, 13, and 31), were transfected into the Freestyle-293F and Cos-1 cells in the same manner as in the case of the EGFP protein expression vector, protein extracts of the supernatant and the cell fraction were obtained, electrophoresis was carried out in 12.5% SDS-PAGE, and a histidine tag at the C terminus of the fusion protein was detected via Western blotting.

### Protein purification

The supernatant was separated from the histidine-tagged proteins released into the FreeStyle 293 medium via centrifugation, the composition of the medium was adjusted at 50 mM Tris HCI (pH = 7.4), 0.5N salt, 10 mM imidazole (Nacalai), and 0.05% Chaps (Dojindo Laboratories), and the resultant was applied to the Ni-NTA Superflow sepharose column (Cat. 30430, Qiagen) to adsorb the histidine-tagged proteins. The column was washed with a buffer containing 50 mM Tris HCl (pH = 7.4), 0.5N salt, 10 mM imidazole, and 0.05% Chaps, washed with a PBS buffer, and then eluted with a buffer containing 0.5N salt and 250 mM imidazole. The eluate was concentrated with the use of an ultrafiltration concentration filter (Amicon Ultra-15, Cat. UFC901024, Millipore) and dialyzed in PBS. The obtained sample was electrophoresed in SDS-PAGE and subjected to Coomassie staining.

Activity of the p53 protein was assayed with the use of the TransAM p53 (Cat. 41196, Active Motif) in accordance with the instructions.

### Results

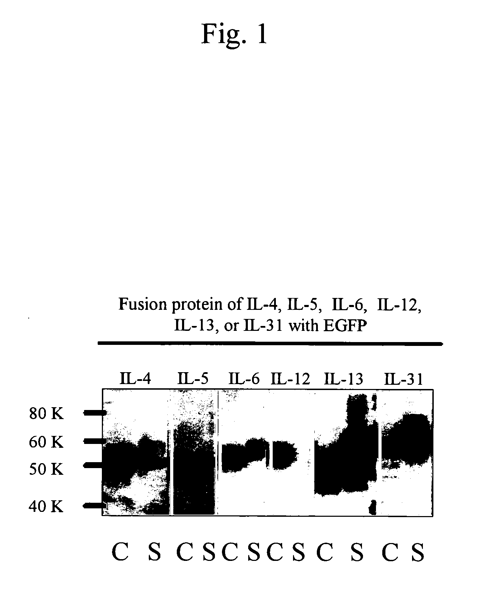

Proteins were expressed in the Freestyle-293F cells with the use of an expression vector carrying polynucleotides encoding the amino acid sequences as shown in SEQ ID NO: 45 (mIL-4-EGFPH protein), SEQ ID NO: 46 (mIL-5-EGFPH protein), SEQ ID NO: 47 (mIL-6-EGFPH protein), SEQ ID NO: 48 (mIL-12-EGFPH protein), SEQ ID NO: 49 (mIL-13-EGFPH protein), and SEQ ID NO: 50 (mIL-31-EGFPH protein), the cell fraction samples and the supernatant fraction samples were subjected to SDS-PAGE electrophoresis, and the expressed proteins were detected via Western blotting with the use of an antibody that recognizes a histidine tag at the C terminus. The results are shown in Fig. 1. In Fig. 1, "C" represents a cell fraction and "S" represents a supernatant fraction. As shown in Fig. 1, interleukin 6 does not have an N-type glycosylation sequence, but proteins, which seem to have experienced sugar chain modification, are released into a supernatant. Fusion proteins of interleukin 13 and interleukin 31 are efficiently released into a supernatant.

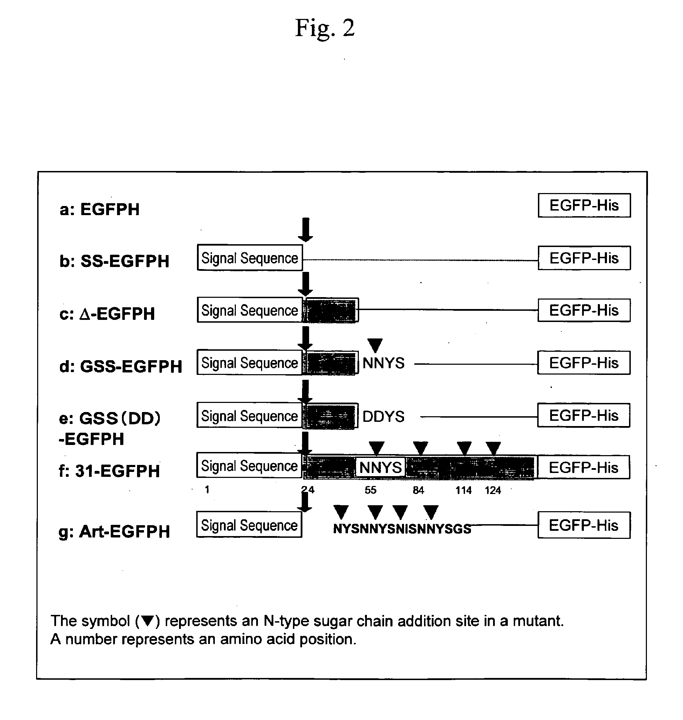

Fig. 2 schematically shows positions of signal sequences and glycosylation sequences in mutants having the amino acid sequences as shown in SEQ ID NO: 44 (EGFP-H), SEQ ID NO: 60 (SS-EGFPH/pcDNA3.1), SEQ ID NO: 61 (Δ-EGFPH/pcDNA3.1), SEQ ID NO: 62 (GSS-EGFPH/pcDNA3.1), SEQ ID NO: 63 (GSS (DD)-EGFPH/pcDNA3.1), SEQ ID NO: 50 (the mIL-31-EGFPH), and SEQ ID NO: 64 (SS-Art-EGFPH/pcDNA3.1). In Fig. 2, a black triangle represents an N-type sugar chain addition site in a mutant and a number represents an amino acid position.

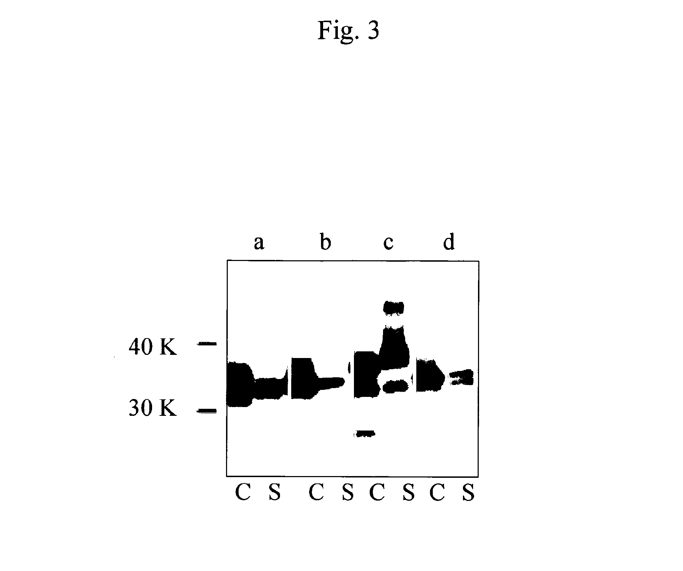

Proteins were expressed in the Freestyle-293F cells with the use of an expression vector carrying polynucleotides encoding the amino acid sequences as shown in SEQ ID NOs: 44, 60, 61, 62, and 63, the cell fraction samples and the supernatant fraction samples were subjected to SDS-PAGE electrophoresis, and the expressed proteins were detected via Western blotting with the use of an antibody that recognizes a histidine tag at the C terminus. The results are shown in Fig. 3. In the lower part of the electrophoresis photograph shown in Fig. 3, "C" represents a cell fraction and "S" represents a supernatant fraction. "a" represents SS-EGFPH (SEQ ID NO: 60), "b" represents Δ-EGFPH (SEQ ID NO: 61), "c" represents GSS-EGFPH (SEQ ID NO: 62), and "d" represents GSS (DD)-EGFPH (SEQ ID NO: 63). As is apparent from Fig. 3, fusion proteins are released into the medium in a particularly efficient manner in the presence of an N-type glycosylation sequence in addition to a signal sequence.

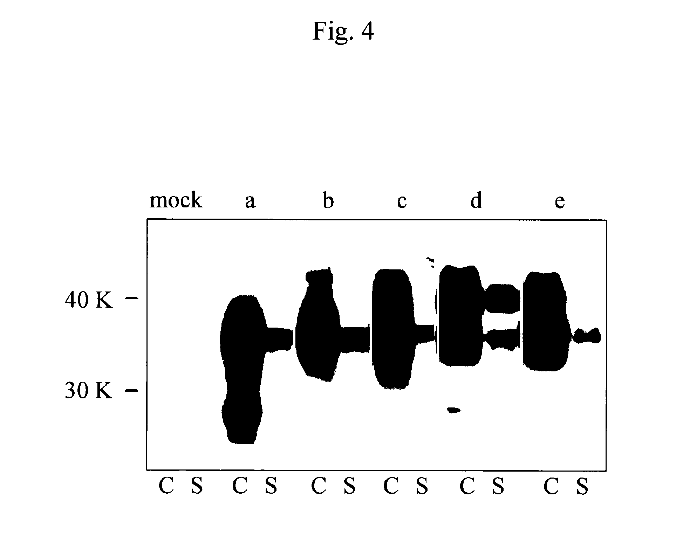

Proteins were expressed in the Cos-1 cells with the use of an expression vector carrying polynucleotides encoding the amino acid sequences as shown in SEQ ID NO: 44, 60, 61, 62, and 63, the cell fraction samples and the supernatant fraction samples were subjected to SDS-PAGE electrophoresis, and the expressed proteins were detected via Western blotting with the use of an antibody that recognizes a histidine tag at the C terminus. The results are shown in Fig. 4. In the lower part of the electrophoresis photograph shown in Fig. 4, "C" represents a cell fraction and "S" represents a supernatant fraction. "a" represents EGFPH (SEQ ID NO: 44), "b" represents SS-EGFPH (SEQ ID NO: 60), "c" represents Δ-EGFPH (SEQ ID NO: 61), "d" represents GSS-EGFPH (SEQ ID NO: 62), and "e" represents GSS (DD)-EGFPH (SEQ ID NO: 63). As shown in Fig. 4, the same situation as in the case shown in Fig. 3 is observed in adhesive cells, as well as in suspension cells.

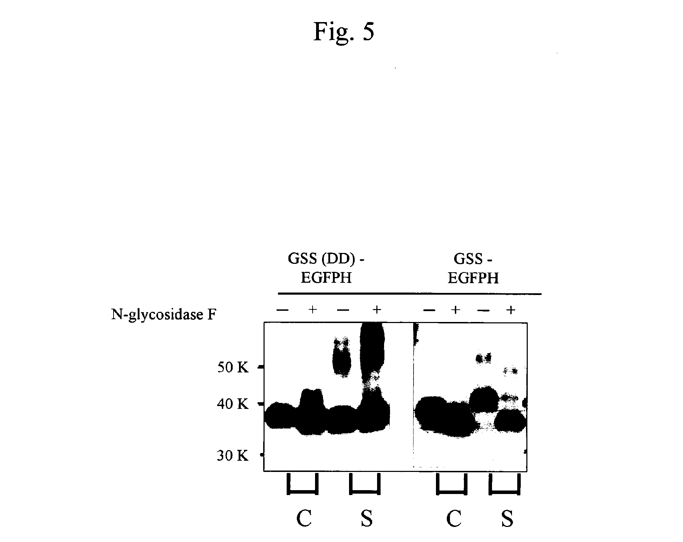

Proteins were expressed in the Freestyle-293F cells with the use of an expression vector carrying polynucleotides encoding the amino acid sequences as shown in SEQ ID NO: 62 (GSS (DD)-EGFPH) and SEQ ID NO: 63 (GSS-EGFPH), the cell fraction samples and the supernatant fraction samples were subjected to denaturation with N-glycosidase F to remove N-type sugar chains, the samples were electrophoresed in 12.5% SDS-PAGE, and a histidine tag at the C terminus was detected via Western blot analysis. The results are shown in Fig. 5. In the lower part of the electrophoresis photograph shown in Fig. 5, "C" represents a cell fraction and "S" represents a supernatant fraction. The results demonstrate that molecular weights of the GSS-EGFPH cell fraction and the GSS-EGFPH supernatant fraction are both lowered as a result of sugar chain removal. The results also demonstrate that the degree of glycosylation in the GSS-EGFPH supernatant fraction is higher than that in the GSS-EGFPH cell fraction. Since both fractions have molecular weights as predicted after sugar chain removal, it is unlikely that other protein modification has taken place.

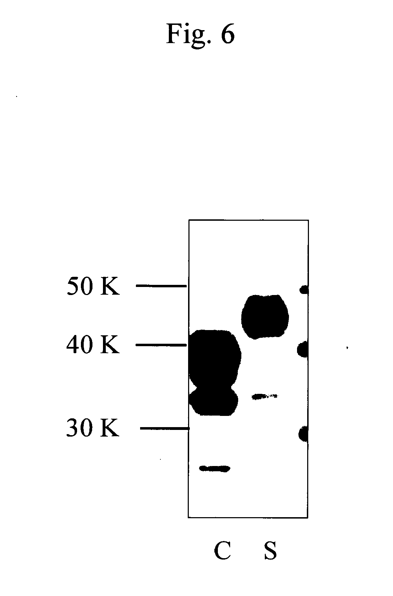

Proteins were expressed in the Freestyle-293F cells with the use of an expression vector carrying a polynucleotide encoding the amino acid sequence as shown in SEQ ID NO: 64 (GSS (ART)-EGFPH), the cell fraction sample and the supernatant fraction sample were subjected to SDS-PAGE electrophoresis, and the expressed proteins were detected via Western blotting with the use of an antibody that recognizes a histidine tag at the C terminus. The results are shown in Fig. 6. In the lower part of the electrophoresis photograph shown in Fig. 6, "C" represents a cell fraction and "S" represents a supernatant fraction. As shown in Fig. 6, sugar chain addition took place and the EGFP proteins were released even with the use of an artificially designed sugar chain modification sequence.

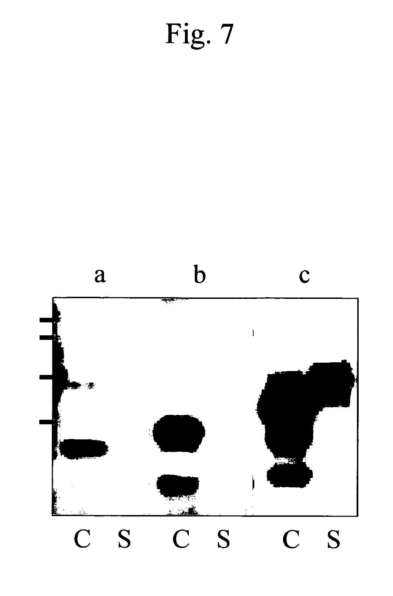

Proteins were expressed in the Freestyle-293F cells with the use of an expression vector carrying polynucleotides encoding the amino acid sequences as shown in SEQ ID NO: 67 (p53H/pcDNA3.1), SEQ ID NO: 68 (SS-p53H/pcDNA3.1), and SEQ ID NO: 69 (GSS-p53H/pcDNA3.1), the cell fraction samples and the supernatant fraction samples were subjected to SDS-PAGE electrophoresis, and the expressed proteins were detected via Western blotting with the use of an antibody that recognizes a histidine tag at the C terminus. The results are shown in Fig. 7. In the lower part of the electrophoresis photograph shown in Fig. 7, "C" represents a cell fraction and "S" represents a supernatant fraction. As shown in Fig. 7, the p53 protein was detected only in a supernatant of the GSS-p53H expression protein to which an endoplasmic reticulum signal sequence and a glycosylation sequence had been added.

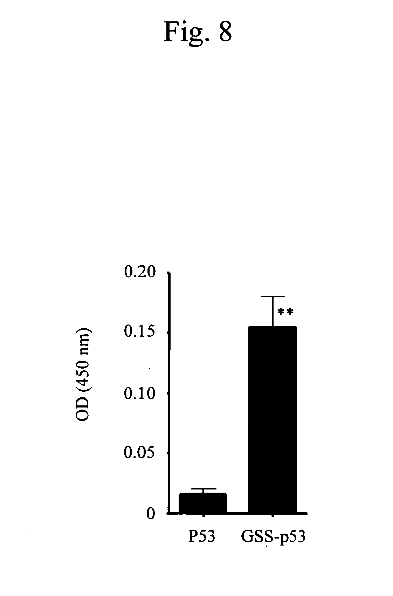

Proteins were expressed in the Freestyle-293F cells with the use of an expression vector carrying polynucleotides encoding the amino acid sequences as shown in SEQ ID NO: 67 (p53) and SEQ ID NO: 69 (GSS-p53), the sequence-selective DNA-binding capacity of the p53 proteins released in the supernatant fraction was assayed with the use of the Trans-AM kit, and the selective DNA-sequence-binding capacity was assayed at the absorption of 450 nm. The results are shown in Fig. 8. Activity of the p53 protein was assayed with the use of the TransAM p53 (Cat. 41196, Active Motif) in accordance with the instructions. As shown in Fig. 8, the active p53 proteins were released to the outside of the cell upon addition of GSS.

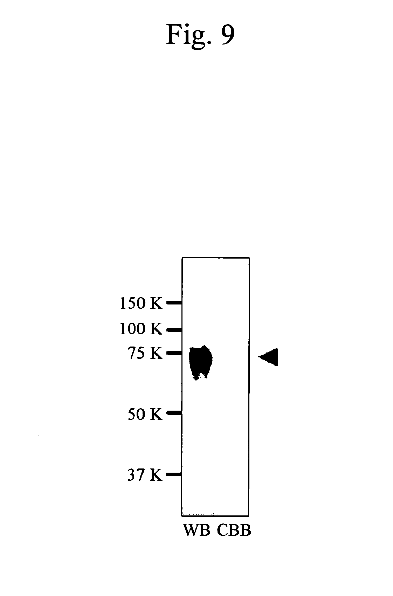

Proteins were expressed in the Freestyle-293F cells with the use of an expression vector carrying a polynucleotide encoding the amino acid sequence as shown in SEQ ID NO: 69, and the supernatant thereof was purified through a nickel chelating column. The results of analysis are shown in Fig. 9.

The supernatant was separated from the histidine-tagged proteins released into the FreeStyle 293 medium via centrifugation, the composition of the medium was adjusted at 50 mM Tris HCI (pH = 7.4), 0.5N salt, 10 mM imidazole (Nacalai), and 0.05% Chaps (Dojindo Laboratories), and the resultant was applied to the Ni-NTA Superflow sepharose column (Cat. 30430, Qiagen) to adsorb the histidine-tagged proteins. The column was washed with a buffer containing 50 mM Tris HCI (pH = 7.4), 0.5N salt, 10 mM imidazole, and 0.05% Chaps, washed with a PBS buffer, and then eluted with a buffer containing 0.5N salt and 250 mM imidazole. The eluate was concentrated with the use of an ultrafiltration concentration filter (Amicon Ultra-15, Cat. UFC901024, Millipore) and dialyzed in PBS. The obtained sample was electrophoresed in SDS-PAGE and subjected to Coomassie staining.

"WB" indicates Western blot analysis conducted with the use of a histidine tag at the C terminus before purification and "CBB" represents an image of the purified protein subjected to Coomassie staining. An arrow head indicates the position of the p53 protein.

As shown in Fig. 9, the released proteins were exclusively purified, which was consistent with the image attained via Western blotting.

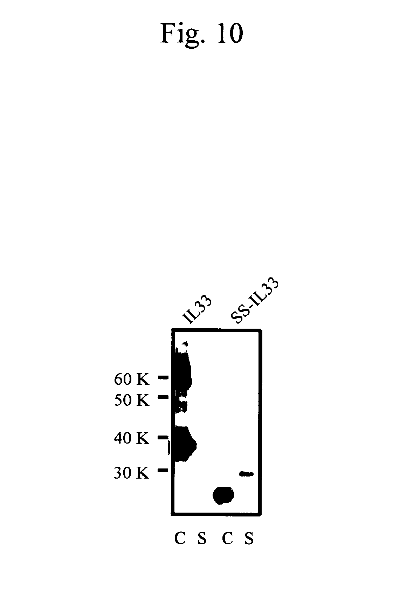

Proteins were expressed in the Freestyle-293F cells with the use of an expression vector carrying polynucleotides encoding the amino acid sequences as shown in SEQ ID NO: 73 (interleukin 33) and SEQ ID NO: 74 (a fusion protein of a signal sequence of interleukin 31 and a mature protein of interleukin 33), the cell fraction samples and the supernatant fraction samples were subjected to SDS-PAGE electrophoresis, and a histidine tag added to the C terminus was detected via Western blotting. The results are shown in Fig. 10. In the lower part of the electrophoresis photograph shown in Fig. 10, "C" represents a cell fraction and "S" represents a supernatant fraction.

As shown in Fig. 10, proteins were not released into the supernatant with the use of the signal sequence of interleukin 33. Upon substitution thereof with the signal sequence of interleukin 31, however, the protein of interleukin 33 was released into the supernatant with a sugar chain being added.

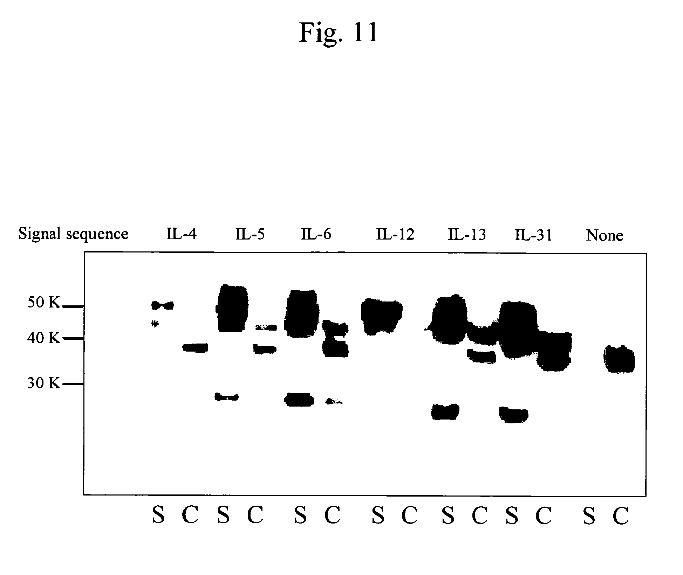

Proteins were expressed in the Freestyle-293F cells with the use of an expression vector carrying polynucleotides encoding the amino acid sequences as shown in SEQ ID NO: 84 (Art-Dsred protein), SEQ ID NO: 90 (mIL4), SEQ ID NO: 91 (mIL5), SEQ ID NO: 92 (mIL6), SEQ ID NO: 93 (mIL12), SEQ ID NO: 94 (mIL13), and SEQ ID NO: 95 (mIL31), the cell fraction samples and the supernatant fraction samples were subjected to SDS-PAGE electrophoresis, and a histidine tag added to the C terminus was detected via Western blotting. The results are shown in Fig. 11. In the lower part of the electrophoresis photograph shown in Fig. 11, "C" represents a cell fraction and "S" represents a supernatant fraction.

Fig. 11 shows a comparison of proteins to be released. Thus, the influence of the signal sequence of each murine interleukin on protein release can be observed. While a protein comprising the amino acid sequence as shown in SEQ ID NO: 84 without a signal sequence was not released, all proteins were efficiently released in the presence of a signal sequence. Also, an artificial glycosylation sequence was found to be effective for extracellular protein release.

### Industrial Applicability

When a polynucleotide encoding a transitional endoplasmic reticulum signal sequence, a polynucleotide encoding an N-type glycosylation sequence consisting of the sequence represented by: Asn-X- (Thr/Ser) (wherein X is an amino acid other than proline) or a polynucleotide encoding an O-type glycosylation sequence, and a polynucleotide encoding a target protein are introduced into eukaryotic host cells, such cells are cultured, and proteins are expressed therein, target proteins, which have experienced sugar chain modification, are efficiently released to the outside of the host cells. By expressing the introduced proteins, which would not be originally released to the outside of the cell, and releasing the expressed proteins to the outside of the cell by such method, target proteins can be efficiently purified. In addition, the efficiency for releasing proteins, which would be originally released, to the outside of the cell can be improved by designing the sequence while taking glycosylation into consideration, and purification or other procedures thereafter can be efficiently carried out.

All publications, patents, and patent applications cited herein are incorporated herein by reference in their entirety.

## Claims

1. A polynucleotide used for producing a recombinant protein in a eukaryotic host cell comprising a polynucleotide encoding a transitional endoplasmic reticulum signal sequence and, in a downstream region thereof, a polynucleotide encoding a fusion protein of a protein with an N-type glycosylation sequence consisting of a sequence represented by: Asn-X-(Thr/Ser) (wherein X is an amino acid other than proline), the polynucleotide being used for releasing the protein to the outside of the eukaryotic host cell.

2. A polynucleotide used for producing a recombinant protein in a eukaryotic host cell comprising a polynucleotide encoding a transitional endoplasmic reticulum signal sequence and, in a downstream region thereof, a polynucleotide encoding a fusion protein of a protein with an O-type glycosylation sequence, the polynucleotide being used for releasing the protein to the outside of the eukaryotic host cell.

3. The polynucleotide according to claim 1 or 2, wherein the transitional endoplasmic reticulum signal sequence is selected from the group consisting of a signal sequence of murine interleukin 4 (SEQ ID NO: 1), a signal sequence of murine interleukin 5 (SEQ ID NO: 3), a signal sequence of murine interleukin 6 (SEQ ID NO: 5), a signal sequence of murine interleukin 12 (SEQ ID NO: 7), a signal sequence of murine interleukin 13 (SEQ ID NO: 9), a signal sequence of murine interleukin 31 (SEQ ID NO: 11), a signal sequence of human interleukin 13(SEQ ID NO: 13), and a signal sequence of human interleukin 31 (SEQ ID NO: 15).

4. An expression vector comprising the polynucleotide according to any of claims 1 to 3, which expresses a recombinant protein and releases the expressed protein to the outside of the eukaryotic host cell.

5. A eukaryotic host cell comprising the expression vector according to claim 4.

6. An expression vector used for producing a recombinant protein in a eukaryotic host cell and for releasing the target protein to the outside of the eukaryotic host cell, which comprises a polynucleotide encoding a transitional endoplasmic reticulum signal sequence, in a downstream region thereof, a polynucleotide encoding an N-type glycosylation sequence consisting of the sequence represented by: Asn-X-(Thr/Ser) (wherein X is an amino acid other than proline), and a multicloning site capable of introducing a foreign gene encoding a target protein to be expressed into a downstream region of the polynucleotide encoding a transitional endoplasmic reticulum signal sequence and an upstream or downstream region of the polynucleotide encoding an N-type glycosylation sequence.

7. An expression vector used for producing a recombinant protein in a eukaryotic host cell and for releasing the target protein to the outside of the host cell, which comprises a polynucleotide encoding a transitional endoplasmic reticulum signal sequence, in a downstream region thereof, a polynucleotide encoding an O-type glycosylation sequence, and a multicloning site capable of introducing a foreign gene encoding a target protein to be expressed into a downstream region of the polynucleotide encoding a transitional endoplasmic reticulum signal sequence and an upstream or downstream region of the polynucleotide encoding an O-type glycosylation sequence.

8. A method for producing a protein comprising introducing the polynucleotide according to any of claims 1 to 3 into a eukaryotic host cell, culturing the eukaryotic host cell, expressing the protein encoded by the polynucleotide, releasing the expressed protein to the outside of the cell, and recovering a target protein from a cell culture supernatant.

9. A method for producing a protein comprising introducing the expression vector according to claim 6 or 7 into a eukaryotic host cell, culturing the eukaryotic host cell, expressing the protein encoded by the polynucleotide, releasing the expressed protein to the outside of the cell, and recovering a target protein from a cell culture supernatant.

10. A protein produced by the method according to claim 8 or 9.

11. The protein according to claim 10, which is subjected to sugar chain modification via addition of an N-type glycosylation sequence consisting of the sequence represented by: Asn-X-(Thr/Ser) (wherein X is an amino acid other than proline) thereto and binding of an N-type sugar chain to the N-type glycosylation sequence.

12. The protein according to claim 11, which is subjected to sugar chain modification via addition of an N-type glycosylation sequence consisting of the sequence represented by: Asn-X-(Thr/Ser) (wherein X is an amino acid other than proline) thereto, the protein not naturally undergoing sugar chain modification, and binding of an N-type sugar chain to the N-type glycosylation sequence.

13. The protein according to claim 10, which is subjected to sugar chain modification via addition of an O-type glycosylation sequence thereto and binding of an O-type sugar chain to the O-type glycosylation sequence.

14. The protein according to claim 13, which is subjected to sugar chain modification via addition of an O-type glycosylation sequence thereto, the protein not naturally undergoing sugar chain modification, and binding of an O-type sugar chain to the O-type glycosylation sequence.

15. A method for producing an antibody via DNA immunization comprising introducing the polynucleotide according to any one of claims 1 to 3 into a non-human animal, expressing a protein encoded by the polynucleotide in the animal body, and producing an antibody against the protein.
